# EUROPEAN PATENT APPLICATION

(11) **EP 2 823 825 A1**
(43) Date of publication of application: **14.01.2015**
(21) Application number: 13758041.1
(22) Date of filing: 06.03.2013
(51) Int. Cl.: A61K 39/395, A61K 31/7105, A61K 48/00, A61P 35/00

(54) **PHARMACEUTICAL COMPOSITION FOR TREATING CANCER**

(30) Priority: 06.03.2012 JP 2012049192
(71) Applicant: Order-Made Medical Research Inc., Noda-shi Chiba 2780022 (JP); National Cancer Center, Tokyo 1040045 (JP)
(72) Inventor: SATOFUKA, Hiroyuki, Noda-shi Chiba 278-0022 (JP); OHSE, Kensuke, Noda-shi Chiba 278-0022 (JP); MUKOUBATA, Shigeki, Noda-shi Chiba 278-0022 (JP); KATO, Yumiko, Noda-shi Chiba 278-0022 (JP); OKABE, Yoko, Noda-shi Chiba 278-0022 (JP); MATSUMURA, Yasuhiro, Kashiwa-shi Chiba 277-8577 (JP); YASUNAGA, Masahiro, Kashiwa-shi Chiba 277-8577 (JP)
(74) Representative: Woods, Geoffrey Corlett
(86) International application number: PCT/JP2013/056884
(87) International publication number: WO 2013/133450

(57) **Abstract**

The object of the present invention is to prepare a cancer therapeutic agent by means of a novel monoclonal antibody which binds to SLC6A6 or its extracellular region and a substance which suppresses SLC6A6 expression.

The present invention provides a pharmaceutical composition comprising a monoclonal antibody which recognizes native SLC6A6 or a polypeptide in the extracellular region of SLC6A6.

SEQUENCE LISTING

<110> Bio Matrix Research, Inc.
National Cancer Center

<120> A pharmaceutical composition for treating a cancer

<130> PCT13-0011

<150> JP 2012-049192
<151> 2012-03-06

<160> 52

<170> PatentIn version 3.5

<210> 1
<211> 1863
<212> DNA
<213> Homo sapiens

<220>
<221> CDS
<222> (1)..(1860)

<400> 1
atg gcc acc aag gag aag ctg cag tgt ctg aaa gat ttc cac aag gac 48
Met Ala Thr Lys Glu Lys Leu Gln Cys Leu Lys Asp Phe His Lys Asp
1               5                   10                  15

atc ctg aag ccc tca cca ggg aag agc cca ggc acg cgg cct gag gac 96
Ile Leu Lys Pro Ser Pro Gly Lys Ser Pro Gly Thr Arg Pro Glu Asp
            20                  25                  30

gag gct gag gga aaa cct ccg cag agg gag aag tgg tct agc aag atc 144
Glu Ala Glu Gly Lys Pro Pro Gln Arg Glu Lys Trp Ser Ser Lys Ile
        35                  40                  45

gac ttt gtg ctc tct gtg gct ggc ggc ttc gtg ggc ttg ggc aac gtc 192
Asp Phe Val Leu Ser Val Ala Gly Gly Phe Val Gly Leu Gly Asn Val
    50                  55                  60

tgg cgc ttc ccg tac ctc tgc tac aag aat ggt gga ggt gcg ttt ctc 240
Trp Arg Phe Pro Tyr Leu Cys Tyr Lys Asn Gly Gly Gly Ala Phe Leu
65                  70                  75                  80

ata ccg tat ttt att ttc ctg ttt ggg agc ggc ctg cct gtg ttt ttc 288
Ile Pro Tyr Phe Ile Phe Leu Phe Gly Ser Gly Leu Pro Val Phe Phe
                85                  90                  95

ttg gag atc atc ata ggc cag tac acc tct gaa ggg ggc atc acc tgc 336
Leu Glu Ile Ile Ile Gly Gln Tyr Thr Ser Glu Gly Gly Ile Thr Cys
            100                 105                 110

tgg gaa aag atc tgc ccc ttg ttc tct ggt atc ggc tat gcc tcc gtt 384
Trp Glu Lys Ile Cys Pro Leu Phe Ser Gly Ile Gly Tyr Ala Ser Val
        115                 120                 125

gta att gtg tcc ctc ctg aat gtc tac tac atc gtc atc ctg gcc tgg 432
Val Ile Val Ser Leu Leu Asn Val Tyr Tyr Ile Val Ile Leu Ala Trp
    130                 135                 140

gcc aca tac tac ctg ttc cag tcc ttc cag aag gag ctg ccc tgg gca 480
Ala Thr Tyr Tyr Leu Phe Gln Ser Phe Gln Lys Glu Leu Pro Trp Ala
145                 150                 155                 160

cac tgc aac cac agc tgg aac aca cct cac tgc atg gag gac acc atg 528
His Cys Asn His Ser Trp Asn Thr Pro His Cys Met Glu Asp Thr Met
                165                 170                 175

cgc aag aac aag agt gtc tgg atc acc atc agc tcc acc aac ttc acc 576
Arg Lys Asn Lys Ser Val Trp Ile Thr Ile Ser Ser Thr Asn Phe Thr
            180                 185                 190

tcc cct gtc atc gag ttc tgg gag cgc aac gtg ctg agc ttg tcc cct 624

Ser Pro Val Ile Glu Phe Trp Glu Arg Asn Val Leu Ser Leu Ser Pro
        195                 200                 205

gga atc gac cac cca ggc tct ctg aaa tgg gac ctc gct ctc tgc ctt 672
Gly Ile Asp His Pro Gly Ser Leu Lys Trp Asp Leu Ala Leu Cys Leu
    210                 215                 220

ctt tta gtc tgg cta gtg tgt ttc ttc tgc atc tgg aag ggc gtc agg 720
Leu Leu Val Trp Leu Val Cys Phe Phe Cys Ile Trp Lys Gly Val Arg
225                 230                 235                 240

tcc act ggg aag gtc gtc tac ttc aca gcc act ttt cca ttc gcc atg 768
Ser Thr Gly Lys Val Val Tyr Phe Thr Ala Thr Phe Pro Phe Ala Met
                245                 250                 255

ctc ctg gtg ctg ctg gtc cga ggg ctg acg ctg ccg ggc gcg ggc gca 816
Leu Leu Val Leu Leu Val Arg Gly Leu Thr Leu Pro Gly Ala Gly Ala
            260                 265                 270

ggc atc aag ttc tat ctg tat cct gac atc acc cgc ctt gag gac cca 864
Gly Ile Lys Phe Tyr Leu Tyr Pro Asp Ile Thr Arg Leu Glu Asp Pro
        275                 280                 285

cag gtg tgg att gac gct ggg act cag ata ttc ttc tct tat gcc atc 912
Gln Val Trp Ile Asp Ala Gly Thr Gln Ile Phe Phe Ser Tyr Ala Ile
    290                 295                 300

tgc ctg ggg gct atg acc tcg ctg ggg agc tac aac aag tac aag tat 960
Cys Leu Gly Ala Met Thr Ser Leu Gly Ser Tyr Asn Lys Tyr Lys Tyr
305                 310                 315                 320

aac tcg tac agg gac tgt atg ctg ctg gga tgc ctg aac agt ggt acc 1008
Asn Ser Tyr Arg Asp Cys Met Leu Leu Gly Cys Leu Asn Ser Gly Thr
                325                 330                 335

agt ttt gtg tct ggc ttc gca att ttt tcc atc ctg ggc ttc atg gca 1056
Ser Phe Val Ser Gly Phe Ala Ile Phe Ser Ile Leu Gly Phe Met Ala
            340                 345                 350

caa gag caa ggg gtg gac att gct gat gtg gct gag tca ggt cct ggc 1104
Gln Glu Gln Gly Val Asp Ile Ala Asp Val Ala Glu Ser Gly Pro Gly
        355                 360                 365

ctg gcc ttc att gcc tac cca aaa gct gtg aca atg atg ccg ctg ccc 1152
Leu Ala Phe Ile Ala Tyr Pro Lys Ala Val Thr Met Met Pro Leu Pro
    370                 375                 380

aca ttt tgg tcc att ctt ttt ttt att atg ctt ctc ttg ctt gga ctg 1200
Thr Phe Trp Ser Ile Leu Phe Phe Ile Met Leu Leu Leu Leu Gly Leu
385                 390                 395                 400

gat agc cag ttt gtt gaa gtt gaa gga cag atc aca tcc ttg gtt gat 1248
Asp Ser Gln Phe Val Glu Val Glu Gly Gln Ile Thr Ser Leu Val Asp
                405                 410                 415

ctt tac cca tcc ttc cta agg aag ggt tat cgt cgg gaa atc ttc atc 1296
Leu Tyr Pro Ser Phe Leu Arg Lys Gly Tyr Arg Arg Glu Ile Phe Ile
            420                 425                 430

gcc ttc gtg tgt agc atc agc tac ctg ctg ggg ctg acg atg gtg acg 1344
Ala Phe Val Cys Ser Ile Ser Tyr Leu Leu Gly Leu Thr Met Val Thr
        435                 440                 445

gag ggt ggc atg tat gtg ttt cag ctc ttt gac tac tat gca gct agc 1392
Glu Gly Gly Met Tyr Val Phe Gln Leu Phe Asp Tyr Tyr Ala Ala Ser
    450                 455                 460

ggt gta tgc ctt ttg tgg gtt gca ttc ttt gaa tgt ttt gtt att gcc 1440
Gly Val Cys Leu Leu Trp Val Ala Phe Phe Glu Cys Phe Val Ile Ala
465                 470                 475                 480

tgg ata tat gga ggt gat aac ctt tat gat ggt att gag gac atg att 1488
Trp Ile Tyr Gly Gly Asp Asn Leu Tyr Asp Gly Ile Glu Asp Met Ile
                485                 490                 495

ggc tat cgg ccc ggg ccc tgg atg aag tac agc tgg gct gtg atc act 1536

Gly Tyr Arg Pro Gly Pro Trp Met Lys Tyr Ser Trp Ala Val Ile Thr
            500                 505                 510

cca gtt ctc tgt gtt gga tgt ttc atc ttc tcg ctc gtc aag tac gta      1584
Pro Val Leu Cys Val Gly Cys Phe Ile Phe Ser Leu Val Lys Tyr Val
        515                 520                 525

ccc ctg acc tac aac aaa aca tac gtg tac ccc aac tgg gcc att ggg      1632
Pro Leu Thr Tyr Asn Lys Thr Tyr Val Tyr Pro Asn Trp Ala Ile Gly
    530                 535                 540

ctg ggc tgg agc ctg gcc ctt tcc tcc atg ctc tgc gtt ccc ttg gtc      1680
Leu Gly Trp Ser Leu Ala Leu Ser Ser Met Leu Cys Val Pro Leu Val
545                 550                 555                 560

atc gtc atc cgc ctc tgc cag act gag ggg ccg ttc ctt gtg aga gtc      1728
Ile Val Ile Arg Leu Cys Gln Thr Glu Gly Pro Phe Leu Val Arg Val
                565                 570                 575

aag tac ctg ctg acc cca agg gaa ccc aac cgc tgg gct gtg gag cgc      1776
Lys Tyr Leu Leu Thr Pro Arg Glu Pro Asn Arg Trp Ala Val Glu Arg
            580                 585                 590

gag gga gcc aca cct tac aac tct cgc acc gtc atg aac ggc gct ctc      1824
Glu Gly Ala Thr Pro Tyr Asn Ser Arg Thr Val Met Asn Gly Ala Leu
        595                 600                 605

gtg aaa ccg acc cac atc att gtg gag acc atg atg tga                  1863
Val Lys Pro Thr His Ile Ile Val Glu Thr Met Met
    610                 615                 620

<210>  2
<211>  620
<212>  PRT
<213>  Homo sapiens

<400>  2

Met Ala Thr Lys Glu Lys Leu Gln Cys Leu Lys Asp Phe His Lys Asp
1               5                   10                  15

Ile Leu Lys Pro Ser Pro Gly Lys Ser Pro Gly Thr Arg Pro Glu Asp
            20                  25                  30

Glu Ala Glu Gly Lys Pro Pro Gln Arg Glu Lys Trp Ser Ser Lys Ile
        35                  40                  45

Asp Phe Val Leu Ser Val Ala Gly Gly Phe Val Gly Leu Gly Asn Val
    50                  55                  60

Trp Arg Phe Pro Tyr Leu Cys Tyr Lys Asn Gly Gly Gly Ala Phe Leu
65                  70                  75                  80

Ile Pro Tyr Phe Ile Phe Leu Phe Gly Ser Gly Leu Pro Val Phe Phe
                85                  90                  95

Leu Glu Ile Ile Ile Gly Gln Tyr Thr Ser Glu Gly Gly Ile Thr Cys
            100                 105                 110

Trp Glu Lys Ile Cys Pro Leu Phe Ser Gly Ile Gly Tyr Ala Ser Val
        115                 120                 125

Val Ile Val Ser Leu Leu Asn Val Tyr Tyr Ile Val Ile Leu Ala Trp
    130                 135                 140

Ala Thr Tyr Tyr Leu Phe Gln Ser Phe Gln Lys Glu Leu Pro Trp Ala

145                 150                 155                 160

His Cys Asn His Ser Trp Asn Thr Pro His Cys Met Glu Asp Thr Met
                165                 170                 175

Arg Lys Asn Lys Ser Val Trp Ile Thr Ile Ser Ser Thr Asn Phe Thr
            180                 185                 190

Ser Pro Val Ile Glu Phe Trp Glu Arg Asn Val Leu Ser Leu Ser Pro
        195                 200                 205

Gly Ile Asp His Pro Gly Ser Leu Lys Trp Asp Leu Ala Leu Cys Leu
    210                 215                 220

Leu Leu Val Trp Leu Val Cys Phe Phe Cys Ile Trp Lys Gly Val Arg
225                 230                 235                 240

Ser Thr Gly Lys Val Val Tyr Phe Thr Ala Thr Phe Pro Phe Ala Met
                245                 250                 255

Leu Leu Val Leu Leu Val Arg Gly Leu Thr Leu Pro Gly Ala Gly Ala
            260                 265                 270

Gly Ile Lys Phe Tyr Leu Tyr Pro Asp Ile Thr Arg Leu Glu Asp Pro
        275                 280                 285

Gln Val Trp Ile Asp Ala Gly Thr Gln Ile Phe Phe Ser Tyr Ala Ile
    290                 295                 300

Cys Leu Gly Ala Met Thr Ser Leu Gly Ser Tyr Asn Lys Tyr Lys Tyr
305                 310                 315                 320

Asn Ser Tyr Arg Asp Cys Met Leu Leu Gly Cys Leu Asn Ser Gly Thr
                325                 330                 335

Ser Phe Val Ser Gly Phe Ala Ile Phe Ser Ile Leu Gly Phe Met Ala
            340                 345                 350

Gln Glu Gln Gly Val Asp Ile Ala Asp Val Ala Glu Ser Gly Pro Gly
        355                 360                 365

Leu Ala Phe Ile Ala Tyr Pro Lys Ala Val Thr Met Met Pro Leu Pro
    370                 375                 380

Thr Phe Trp Ser Ile Leu Phe Phe Ile Met Leu Leu Leu Leu Gly Leu
385                 390                 395                 400

Asp Ser Gln Phe Val Glu Val Glu Gly Gln Ile Thr Ser Leu Val Asp
                405                 410                 415

Leu Tyr Pro Ser Phe Leu Arg Lys Gly Tyr Arg Arg Glu Ile Phe Ile
            420                 425                 430

Ala Phe Val Cys Ser Ile Ser Tyr Leu Leu Gly Leu Thr Met Val Thr
        435                 440                 445

Glu Gly Gly Met Tyr Val Phe Gln Leu Phe Asp Tyr Tyr Ala Ala Ser

450                 455                 460

Gly Val Cys Leu Leu Trp Val Ala Phe Phe Glu Cys Phe Val Ile Ala
465                 470                 475                 480

Trp Ile Tyr Gly Gly Asp Asn Leu Tyr Asp Gly Ile Glu Asp Met Ile
                485                 490                 495

Gly Tyr Arg Pro Gly Pro Trp Met Lys Tyr Ser Trp Ala Val Ile Thr
            500                 505                 510

Pro Val Leu Cys Val Gly Cys Phe Ile Phe Ser Leu Val Lys Tyr Val
        515                 520                 525

Pro Leu Thr Tyr Asn Lys Thr Tyr Val Tyr Pro Asn Trp Ala Ile Gly
    530                 535                 540

Leu Gly Trp Ser Leu Ala Leu Ser Ser Met Leu Cys Val Pro Leu Val
545                 550                 555                 560

Ile Val Ile Arg Leu Cys Gln Thr Glu Gly Pro Phe Leu Val Arg Val
                565                 570                 575

Lys Tyr Leu Leu Thr Pro Arg Glu Pro Asn Arg Trp Ala Val Glu Arg
            580                 585                 590

Glu Gly Ala Thr Pro Tyr Asn Ser Arg Thr Val Met Asn Gly Ala Leu
        595                 600                 605

Val Lys Pro Thr His Ile Ile Val Glu Thr Met Met
    610                 615                 620

<210>  3
<211>  222
<212>  DNA
<213>  Homo sapiens

<220>
<221>  CDS
<222>  (1)..(222)

<400>  3
gcc tgg gcc aca tac tac ctg ttc cag tcc ttc cag aag gag ctg ccc          48
Ala Trp Ala Thr Tyr Tyr Leu Phe Gln Ser Phe Gln Lys Glu Leu Pro
1               5                   10                  15

tgg gca cac tgc aac cac agc tgg aac aca cct cac tgc atg gag gac          96
Trp Ala His Cys Asn His Ser Trp Asn Thr Pro His Cys Met Glu Asp
            20                  25                  30

acc atg cgc aag aac aag agt gtc tgg atc acc atc agc tcc acc aac          144
Thr Met Arg Lys Asn Lys Ser Val Trp Ile Thr Ile Ser Ser Thr Asn
        35                  40                  45

ttc acc tcc cct gtc atc gag ttc tgg gag cgc aac gtg ctg agc ttg          192
Phe Thr Ser Pro Val Ile Glu Phe Trp Glu Arg Asn Val Leu Ser Leu
    50                  55                  60

tcc cct gga atc gac cac cca ggc tct ctg                                  222
Ser Pro Gly Ile Asp His Pro Gly Ser Leu
65                  70

<210>  4

<211>  74
<212>  PRT
<213>  Homo sapiens

<400>  4

Ala Trp Ala Thr Tyr Tyr Leu Phe Gln Ser Phe Gln Lys Glu Leu Pro
1               5                   10                  15

Trp Ala His Cys Asn His Ser Trp Asn Thr Pro His Cys Met Glu Asp
            20                  25                  30

Thr Met Arg Lys Asn Lys Ser Val Trp Ile Thr Ile Ser Ser Thr Asn
        35                  40                  45

Phe Thr Ser Pro Val Ile Glu Phe Trp Glu Arg Asn Val Leu Ser Leu
    50                  55                  60

Ser Pro Gly Ile Asp His Pro Gly Ser Leu
65                  70

<210>  5
<211>  31
<212>  DNA
<213>  Artificial

<220>
<223>  Synthetic DNA

<400>  5
aaaggatcca tggccaccaa ggagaagctg c                                    31

<210> 6
<211> 33
<212> DNA
<213> Artificial

<220>
<223> Synthetic DNA

<400> 6
aaatctagac atcatggtct ccacaatgat gtg 33

<210> 7
<211> 31
<212> DNA
<213> Artificial

<220>
<223> Synthetic DNA

<400> 7
ataggatccg gcctgggcca catatcacct g 31

<210> 8
<211> 32
<212> DNA
<213> Artificial

<220>
<223> Synthetic DNA

<400> 8
tatgaattcg ctttcagaga gcctgggtgg tc 32

<210> 9
<211> 22

<212>  DNA
<213>  Artificial

<220>
<223>  Synthetic DNA

<400>  9
aagtsmarct gcagsagtcw gg                                                22

<210>  10
<211>  39
<212>  DNA
<213>  Artificial

<220>
<223>  Synthetic DNA

<400>  10
ggagacgagg gggaaaagct ttgggaagga ctgactctc                              39

<210>  11
<211>  24
<212>  DNA
<213>  Artificial

<220>
<223>  Synthetic DNA

<400>  11
gacattcagc tgacccagtc tcca                                              24

<210>  12
<211>  22
<212>  DNA
<213>  Artificial

<220>
<223> Synthetic DNA

<400> 12
gttagatctc cagcttggtc cc 22

<210> 13
<211> 22
<212> DNA
<213> Artificial

<220>
<223> Synthetic DNA

<400> 13
atggcttggg tgtggacctt gc 22

<210> 14
<211> 41
<212> DNA
<213> Artificial

<220>
<223> Synthetic DNA

<400> 14
ccagactgct gcagtttgac ctgtgcttgg gcactttggg c 41

<210> 15
<211> 42
<212> DNA
<213> Artificial

<220>

<223>  Synthetic DNA

<400>  15
ctctggtcac tgtctctgca gccaaaacga cacccccatc tg                42

<210>  16
<211>  24
<212>  DNA
<213>  Artificial

<220>
<223>  Synthetic DNA

<400>  16
tcatttacca ggagagtggg agag                                    24

<210>  17
<211>  39
<212>  DNA
<213>  Artificial

<220>
<223>  Synthetic DNA

<400>  17
ggcttcatgg tgctcagtgt gacattcagc tgacccagt                    39

<210>  18
<211>  40
<212>  DNA
<213>  Artificial

<220>
<223>  Synthetic DNA

<400> 18
agttggtgca gcatcagccc gtttgatctc cagcttggtc 40

<210> 19
<211> 40
<212> DNA
<213> Artificial

<220>
<223> Synthetic DNA

<400> 19
gaccaagctg gagatcaaac gggctgatgc tgcaccaact 40

<210> 20
<211> 31
<212> DNA
<213> Artificial

<220>
<223> Synthetic DNA

<400> 20
tttgaattcc taacactcat tcctgttgaa g 31

<210> 21
<211> 1392
<212> DNA
<213> Homo sapiens

<220>
<221> CDS
<222> (1)..(1389)

<400> 21
atg gct tgg gtg tgg acc ttg cta ctc ctg atg gca gct gcc caa agt 48
Met Ala Trp Val Trp Thr Leu Leu Leu Leu Met Ala Ala Ala Gln Ser
1               5                   10                  15

gcc caa gca cag gtc aaa ctg cag cag tct gga gct gag ctg atg aag 96
Ala Gln Ala Gln Val Lys Leu Gln Gln Ser Gly Ala Glu Leu Met Lys
            20                  25                  30

cct ggg gcc tca gtg aag ata tcc tgc aag gct act ggc tac aca ttc 144
Pro Gly Ala Ser Val Lys Ile Ser Cys Lys Ala Thr Gly Tyr Thr Phe
        35                  40                  45

agt agg tac tgg ata gag tgg gta aag cag agg cct gga cat ggc ctt 192
Ser Arg Tyr Trp Ile Glu Trp Val Lys Gln Arg Pro Gly His Gly Leu
    50                  55                  60

gag tgg att gga gag att tta cct gga agt ggt agt act aac tac aat 240
Glu Trp Ile Gly Glu Ile Leu Pro Gly Ser Gly Ser Thr Asn Tyr Asn
65                  70                  75                  80

gag aag ttc aag ggc aag gcc aca ttc act gca gat aca tcc tcc aac 288
Glu Lys Phe Lys Gly Lys Ala Thr Phe Thr Ala Asp Thr Ser Ser Asn
                85                  90                  95

aca gcc tac atg caa ctc agc agc ctg aca tct gag gac tct gcc gtc 336
Thr Ala Tyr Met Gln Leu Ser Ser Leu Thr Ser Glu Asp Ser Ala Val
            100                 105                 110

tat tac tgt gca aga gga gga ttt ctt ggg cat tac ggg ttt gct tac 384
Tyr Tyr Cys Ala Arg Gly Gly Phe Leu Gly His Tyr Gly Phe Ala Tyr
        115                 120                 125

tgg ggc caa ggg act ctg gtc act gtc tct gca gcc aaa acg aca ccc 432
Trp Gly Gln Gly Thr Leu Val Thr Val Ser Ala Ala Lys Thr Thr Pro
    130                 135                 140

cca tct gtc tat cca ctg gcc cct gga tct gct gcc caa act aac tcc 480

Pro Ser Val Tyr Pro Leu Ala Pro Gly Ser Ala Ala Gln Thr Asn Ser
145                 150                 155                 160

atg gtg acc ctg gga tgc ctg gtc aag ggc tat ttc cct gag cca gtg 528
Met Val Thr Leu Gly Cys Leu Val Lys Gly Tyr Phe Pro Glu Pro Val
                165                 170                 175

aca gtg acc tgg aac tct gga tcc ctg tcc agc ggt gtg cac acc ttc 576
Thr Val Thr Trp Asn Ser Gly Ser Leu Ser Ser Gly Val His Thr Phe
            180                 185                 190

cca gct gtc ctg cag tct gac ctc tac act ctg agc agc tca gtg act 624
Pro Ala Val Leu Gln Ser Asp Leu Tyr Thr Leu Ser Ser Ser Val Thr
        195                 200                 205

gtc ccc tcc agc acc tgg ccc agc gag acc gtc acc tgc aac gtt gcc 672
Val Pro Ser Ser Thr Trp Pro Ser Glu Thr Val Thr Cys Asn Val Ala
    210                 215                 220

cac ccg gcc agc agc acc aag gtg gac aag aaa att gtg ccc agg gat 720
His Pro Ala Ser Ser Thr Lys Val Asp Lys Lys Ile Val Pro Arg Asp
225                 230                 235                 240

tgt ggt tgt aag cct tgc ata tgt aca gtc cca gaa gta tca tct gtc 768
Cys Gly Cys Lys Pro Cys Ile Cys Thr Val Pro Glu Val Ser Ser Val
                245                 250                 255

ttc atc ttc ccc cca aag ccc aag gat gtg ctc acc att act ctg act 816
Phe Ile Phe Pro Pro Lys Pro Lys Asp Val Leu Thr Ile Thr Leu Thr
            260                 265                 270

cct aag gtc acg tgt gtt gtg gta gac atc agc aag gat gat ccc gag 864
Pro Lys Val Thr Cys Val Val Val Asp Ile Ser Lys Asp Asp Pro Glu
        275                 280                 285

gtc cag ttc agc tgg ttt gta gat gat gtg gag gtg cac aca gct cag 912
Val Gln Phe Ser Trp Phe Val Asp Asp Val Glu Val His Thr Ala Gln
    290                 295                 300

acg caa ccc cgg gag gag cag ttc aac agc act ttc cgc tca gtc agt 960
Thr Gln Pro Arg Glu Glu Gln Phe Asn Ser Thr Phe Arg Ser Val Ser
305                 310                 315                 320

gaa ctt ccc atc atg cac cag gac tgg ctc aat ggc aag gag ttc aaa 1008
Glu Leu Pro Ile Met His Gln Asp Trp Leu Asn Gly Lys Glu Phe Lys
                325                 330                 335

tgc agg gtc aac agt gca gct ttc cct gcc ccc atc gag aaa acc atc 1056
Cys Arg Val Asn Ser Ala Ala Phe Pro Ala Pro Ile Glu Lys Thr Ile
            340                 345                 350

tcc aaa acc aaa ggc aga ccg aag gct cca cag gtg tac acc att cca 1104
Ser Lys Thr Lys Gly Arg Pro Lys Ala Pro Gln Val Tyr Thr Ile Pro
        355                 360                 365

cct ccc aag gag cag atg gcc aag gat aaa gtc agt ctg acc tgc atg 1152
Pro Pro Lys Glu Gln Met Ala Lys Asp Lys Val Ser Leu Thr Cys Met
    370                 375                 380

ata aca gac ttc ttc cct gaa gac att act gtg gag tgg cag tgg aat 1200
Ile Thr Asp Phe Phe Pro Glu Asp Ile Thr Val Glu Trp Gln Trp Asn
385                 390                 395                 400

ggg cag cca gcg gag aac tac aag aac act cag ccc atc atg gac aca 1248
Gly Gln Pro Ala Glu Asn Tyr Lys Asn Thr Gln Pro Ile Met Asp Thr
                405                 410                 415

gat ggc tct tac ttc gtc tac agc aag ctc aat gtg cag aag agc aac 1296
Asp Gly Ser Tyr Phe Val Tyr Ser Lys Leu Asn Val Gln Lys Ser Asn
            420                 425                 430

tgg gag gca gga aat act ttc acc tgc tct gtg tta cat gag ggc ctg 1344
Trp Glu Ala Gly Asn Thr Phe Thr Cys Ser Val Leu His Glu Gly Leu
        435                 440                 445

cac aac cac cat act gag aag agc ctc tcc cac tct cct ggt aaa tga 1392

His Asn His His Thr Glu Lys Ser Leu Ser His Ser Pro Gly Lys
    450                 455                 460

<210>  22
<211>  463
<212>  PRT
<213>  Homo sapiens

<400>  22

Met Ala Trp Val Trp Thr Leu Leu Leu Leu Met Ala Ala Ala Gln Ser
1               5                   10                  15

Ala Gln Ala Gln Val Lys Leu Gln Gln Ser Gly Ala Glu Leu Met Lys
            20                  25                  30

Pro Gly Ala Ser Val Lys Ile Ser Cys Lys Ala Thr Gly Tyr Thr Phe
        35                  40                  45

Ser Arg Tyr Trp Ile Glu Trp Val Lys Gln Arg Pro Gly His Gly Leu
    50                  55                  60

Glu Trp Ile Gly Glu Ile Leu Pro Gly Ser Gly Ser Thr Asn Tyr Asn
65                  70                  75                  80

Glu Lys Phe Lys Gly Lys Ala Thr Phe Thr Ala Asp Thr Ser Ser Asn
                85                  90                  95

Thr Ala Tyr Met Gln Leu Ser Ser Leu Thr Ser Glu Asp Ser Ala Val
            100                 105                 110

Tyr Tyr Cys Ala Arg Gly Gly Phe Leu Gly His Tyr Gly Phe Ala Tyr
        115                 120                 125

Trp Gly Gln Gly Thr Leu Val Thr Val Ser Ala Ala Lys Thr Thr Pro
    130                 135                 140

Pro Ser Val Tyr Pro Leu Ala Pro Gly Ser Ala Ala Gln Thr Asn Ser
145                 150                 155                 160

Met Val Thr Leu Gly Cys Leu Val Lys Gly Tyr Phe Pro Glu Pro Val
                165                 170                 175

Thr Val Thr Trp Asn Ser Gly Ser Leu Ser Ser Gly Val His Thr Phe
            180                 185                 190

Pro Ala Val Leu Gln Ser Asp Leu Tyr Thr Leu Ser Ser Ser Val Thr
        195                 200                 205

Val Pro Ser Ser Thr Trp Pro Ser Glu Thr Val Thr Cys Asn Val Ala
    210                 215                 220

His Pro Ala Ser Ser Thr Lys Val Asp Lys Lys Ile Val Pro Arg Asp
225                 230                 235                 240

Cys Gly Cys Lys Pro Cys Ile Cys Thr Val Pro Glu Val Ser Ser Val
                245                 250                 255

Phe Ile Phe Pro Pro Lys Pro Lys Asp Val Leu Thr Ile Thr Leu Thr

            260                 265                 270

Pro Lys Val Thr Cys Val Val Val Asp Ile Ser Lys Asp Asp Pro Glu
        275                 280                 285

Val Gln Phe Ser Trp Phe Val Asp Asp Val Glu Val His Thr Ala Gln
    290                 295                 300

Thr Gln Pro Arg Glu Glu Gln Phe Asn Ser Thr Phe Arg Ser Val Ser
305                 310                 315                 320

Glu Leu Pro Ile Met His Gln Asp Trp Leu Asn Gly Lys Glu Phe Lys
                325                 330                 335

Cys Arg Val Asn Ser Ala Ala Phe Pro Ala Pro Ile Glu Lys Thr Ile
            340                 345                 350

Ser Lys Thr Lys Gly Arg Pro Lys Ala Pro Gln Val Tyr Thr Ile Pro
        355                 360                 365

Pro Pro Lys Glu Gln Met Ala Lys Asp Lys Val Ser Leu Thr Cys Met
    370                 375                 380

Ile Thr Asp Phe Phe Pro Glu Asp Ile Thr Val Glu Trp Gln Trp Asn
385                 390                 395                 400

Gly Gln Pro Ala Glu Asn Tyr Lys Asn Thr Gln Pro Ile Met Asp Thr
                405                 410                 415

Asp Gly Ser Tyr Phe Val Tyr Ser Lys Leu Asn Val Gln Lys Ser Asn
            420                 425                 430

Trp Glu Ala Gly Asn Thr Phe Thr Cys Ser Val Leu His Glu Gly Leu
        435                 440                 445

His Asn His His Thr Glu Lys Ser Leu Ser His Ser Pro Gly Lys
    450                 455                 460

<210> 23
<211> 708
<212> DNA
<213> Homo sapiens

<220>
<221> CDS
<222> (1)..(705)

<400> 23
atg aga ccg tct att cag ttc ctg ggg ctc ttg ttg ttc tgg ctt cat 48
Met Arg Pro Ser Ile Gln Phe Leu Gly Leu Leu Leu Phe Trp Leu His
1               5                   10                  15

ggt gct cag tgt gac att cag ctg acc cag tct cca acc acc atg gct 96
Gly Ala Gln Cys Asp Ile Gln Leu Thr Gln Ser Pro Thr Thr Met Ala
            20                  25                  30

gca tct ccc ggg gag aag atc act atc acc tgc agt gcc agc tca agt 144
Ala Ser Pro Gly Glu Lys Ile Thr Ile Thr Cys Ser Ala Ser Ser Ser
        35                  40                  45

ata agt tcc aat tac ttg cat tgg tat cag cag aag cca gga ttc tcc 192
Ile Ser Ser Asn Tyr Leu His Trp Tyr Gln Gln Lys Pro Gly Phe Ser

    50                  55                  60

cct aaa ctc ttg att tat agg aca tcc aat ctg gct tct gga gtc cca 240
Pro Lys Leu Leu Ile Tyr Arg Thr Ser Asn Leu Ala Ser Gly Val Pro
65                  70                  75                  80

gct cgc ttc agt ggc agt ggg tct ggg acc tct tac tct ctc aca att 288
Ala Arg Phe Ser Gly Ser Gly Ser Gly Thr Ser Tyr Ser Leu Thr Ile
                85                  90                  95

ggc acc atg gag gct gaa gat gtt gcc act tac tac tgc cag cag ggt 336
Gly Thr Met Glu Ala Glu Asp Val Ala Thr Tyr Tyr Cys Gln Gln Gly
            100                 105                 110

agt agt ata ccg tac acg ttc gga ggg ggg acc aag ctg gag atc aaa 384
Ser Ser Ile Pro Tyr Thr Phe Gly Gly Gly Thr Lys Leu Glu Ile Lys
        115                 120                 125

cgg gct gat gct gca cca act gta tcc atc ttc cca cca tcc agt gag 432
Arg Ala Asp Ala Ala Pro Thr Val Ser Ile Phe Pro Pro Ser Ser Glu
    130                 135                 140

cag tta aca tct gga ggt gcc tca gtc gtg tgc ttc ttg aac aac ttc 480
Gln Leu Thr Ser Gly Gly Ala Ser Val Val Cys Phe Leu Asn Asn Phe
145                 150                 155                 160

tac ccc aaa gac atc aat gtc aag tgg aag att gat ggc agt gaa cga 528
Tyr Pro Lys Asp Ile Asn Val Lys Trp Lys Ile Asp Gly Ser Glu Arg
                165                 170                 175

caa aat ggc gtc ctg aac agt tgg act gat cag gac agc aaa gac agc 576
Gln Asn Gly Val Leu Asn Ser Trp Thr Asp Gln Asp Ser Lys Asp Ser
            180                 185                 190

acc tac agc atg agc agc acc ctc acg ttg acc aag gac gag tat gaa 624
Thr Tyr Ser Met Ser Ser Thr Leu Thr Leu Thr Lys Asp Glu Tyr Glu
        195                 200                 205

cga cat aac agc tat acc tgt gag gcc act cac aag aca tca act tca     672
Arg His Asn Ser Tyr Thr Cys Glu Ala Thr His Lys Thr Ser Thr Ser
    210                 215                 220

ccc att gtc aag agc ttc aac agg aat gag tgt tag                     708
Pro Ile Val Lys Ser Phe Asn Arg Asn Glu Cys
225                 230                 235

<210>  24
<211>  235
<212>  PRT
<213>  Homo sapiens

<400>  24

Met Arg Pro Ser Ile Gln Phe Leu Gly Leu Leu Leu Phe Trp Leu His
1               5                   10                  15

Gly Ala Gln Cys Asp Ile Gln Leu Thr Gln Ser Pro Thr Thr Met Ala
            20                  25                  30

Ala Ser Pro Gly Glu Lys Ile Thr Ile Thr Cys Ser Ala Ser Ser Ser
        35                  40                  45

Ile Ser Ser Asn Tyr Leu His Trp Tyr Gln Gln Lys Pro Gly Phe Ser
    50                  55                  60

Pro Lys Leu Leu Ile Tyr Arg Thr Ser Asn Leu Ala Ser Gly Val Pro
65                  70                  75                  80

Ala Arg Phe Ser Gly Ser Gly Ser Gly Thr Ser Tyr Ser Leu Thr Ile
                85                  90                  95

Gly Thr Met Glu Ala Glu Asp Val Ala Thr Tyr Tyr Cys Gln Gln Gly
            100                 105                 110

Ser Ser Ile Pro Tyr Thr Phe Gly Gly Gly Thr Lys Leu Glu Ile Lys
        115                 120                 125

Arg Ala Asp Ala Ala Pro Thr Val Ser Ile Phe Pro Pro Ser Ser Glu
    130                 135                 140

Gln Leu Thr Ser Gly Gly Ala Ser Val Val Cys Phe Leu Asn Asn Phe
145                 150                 155                 160

Tyr Pro Lys Asp Ile Asn Val Lys Trp Lys Ile Asp Gly Ser Glu Arg
                165                 170                 175

Gln Asn Gly Val Leu Asn Ser Trp Thr Asp Gln Asp Ser Lys Asp Ser
            180                 185                 190

Thr Tyr Ser Met Ser Ser Thr Leu Thr Leu Thr Lys Asp Glu Tyr Glu
        195                 200                 205

Arg His Asn Ser Tyr Thr Cys Glu Ala Thr His Lys Thr Ser Thr Ser
    210                 215                 220

Pro Ile Val Lys Ser Phe Asn Arg Asn Glu Cys
225                 230                 235

<210> 25
<211> 41
<212> DNA
<213> Artificial

<220>
<223> Synthetic DNA

<400> 25
gcccaaagtg cccaagcaca ggtcaaactg cagcagtctg g 41

<210> 26
<211> 40
<212> DNA
<213> Artificial

<220>
<223> Synthetic DNA

<400> 26
gatgggccct tggtgctagc tgcagagaca gtgaccagag 40

<210> 27
<211> 41
<212> DNA
<213> Artificial

<220>
<223> Synthetic DNA

<400> 27
ctctggtcac tgtctctgca gctagcacca agggcccatc g 41

<210> 28
<211> 31

<212>  DNA
<213>  Artificial

<220>
<223>  Synthetic DNA

<400>  28
tttgcggccg ctcatttacc cggagacagg g                                    31


<210>  29
<211>  34
<212>  DNA
<213>  Artificial

<220>
<223>  Synthetic DNA

<400>  29
tttgcggccg caccatggct tgggtgtgga cctt                                 34


<210>  30
<211>  41
<212>  DNA
<213>  Artificial

<220>
<223>  Synthetic DNA

<400>  30
ccagactgct gcagtttgac ctgtgcttgg gcactttggg c                         41


<210>  31
<211>  40
<212>  DNA
<213>  Artificial

<220>
<223>  Synthetic DNA

<400>  31
ggcttcatgg tgctcagtgt gacattcagc tgacccagtc                                40

<210>  32
<211>  40
<212>  DNA
<213>  Artificial

<220>
<223>  Synthetic DNA

<400>  32
agatggtgca gccaccgtac gtttgatctc cagcttggtc                                40

<210>  33
<211>  40
<212>  DNA
<213>  Artificial

<220>
<223>  Synthetic DNA

<400>  33
gaccaagctg gagatcaaac gtacggtggc tgcaccatct                                40

<210>  34
<211>  31
<212>  DNA
<213>  Artificial

<220>

<223>  Synthetic DNA

<400>  34
tttgaattct aacactctcc cctgttgaag c                                    31

<210>  35
<211>  32
<212>  DNA
<213>  Artificial

<220>
<223>  Synthetic DNA

<400>  35
tttggtacca ccatgagacc gtctattcag tt                                   32

<210>  36
<211>  40
<212>  DNA
<213>  Artificial

<220>
<223>  Synthetic DNA

<400>  36
gactgggtca gctgaatgtc acactgagca ccatgaagcc                           40

<210>  37
<211>  1131
<212>  DNA
<213>  Homo sapiens

<220>
<221>  CDS

<222>  (1)..(1128)

<400>  37
tcc tcc aac aca gcc tac atg caa ctc agc agc ctg aca tct gag gac      48
Ser Ser Asn Thr Ala Tyr Met Gln Leu Ser Ser Leu Thr Ser Glu Asp
1               5                   10                  15

tct gcc gtc tat tac tgt gca aga gga gga ttt ctt ggg cat tac ggg      96
Ser Ala Val Tyr Tyr Cys Ala Arg Gly Gly Phe Leu Gly His Tyr Gly
            20                  25                  30

ttt gct tac tgg ggc caa ggg act ctg gtc act gtc tct gca gct agc      144
Phe Ala Tyr Trp Gly Gln Gly Thr Leu Val Thr Val Ser Ala Ala Ser
        35                  40                  45

acc aag ggc cca tcg gtc ttc ccc ctg gca ccc tcc tcc aag agc acc      192
Thr Lys Gly Pro Ser Val Phe Pro Leu Ala Pro Ser Ser Lys Ser Thr
    50                  55                  60

tct ggg ggc aca gcg gcc ctg ggc tgc ctg gtc aag gac tac ttc ccc      240
Ser Gly Gly Thr Ala Ala Leu Gly Cys Leu Val Lys Asp Tyr Phe Pro
65                  70                  75                  80

gaa ccg gtg acg gtg tcg tgg aac tca ggc gcc ctg acc agc ggc gtg      288
Glu Pro Val Thr Val Ser Trp Asn Ser Gly Ala Leu Thr Ser Gly Val
                85                  90                  95

cac acc ttc ccg gct gtc cta cag tcc tca gga ctc tac tcc ctc agc      336
His Thr Phe Pro Ala Val Leu Gln Ser Ser Gly Leu Tyr Ser Leu Ser
            100                 105                 110

agc gtg gtg acc gtg ccc tcc agc agc ttg ggc acc cag acc tac atc      384
Ser Val Val Thr Val Pro Ser Ser Ser Leu Gly Thr Gln Thr Tyr Ile
        115                 120                 125

tgc aac gtg aat cac aag ccc agc aac acc aag gtg gac aag aaa gtt      432
Cys Asn Val Asn His Lys Pro Ser Asn Thr Lys Val Asp Lys Lys Val
    130                 135                 140

gag ccc aaa tct tgt gac aaa act cac aca tgc cca ccg tgc cca gca 480
Glu Pro Lys Ser Cys Asp Lys Thr His Thr Cys Pro Pro Cys Pro Ala
145                 150                 155                 160

cct gaa ctc ctg ggg gga ccg tca gtc ttc ctc ttc ccc cca aaa ccc 528
Pro Glu Leu Leu Gly Gly Pro Ser Val Phe Leu Phe Pro Pro Lys Pro
                165                 170                 175

aag gac acc ctc atg atc tcc cgg acc cct gag gtc aca tgc gtg gtg 576
Lys Asp Thr Leu Met Ile Ser Arg Thr Pro Glu Val Thr Cys Val Val
            180                 185                 190

gtg gac gtg agc cac gaa gac cct gag gtc aag ttc aac tgg tac gtg 624
Val Asp Val Ser His Glu Asp Pro Glu Val Lys Phe Asn Trp Tyr Val
        195                 200                 205

gac ggc gtg gag gtg cat aat gcc aag aca aag ccg cgg gag gag cag 672
Asp Gly Val Glu Val His Asn Ala Lys Thr Lys Pro Arg Glu Glu Gln
    210                 215                 220

tac aac agc acg tac cgt gtg gtc agc gtc ctc acc gtc ctg cac cag 720
Tyr Asn Ser Thr Tyr Arg Val Val Ser Val Leu Thr Val Leu His Gln
225                 230                 235                 240

gac tgg ctg aat ggc aag gag tac aag tgc aag gtc tcc aac aaa gcc 768
Asp Trp Leu Asn Gly Lys Glu Tyr Lys Cys Lys Val Ser Asn Lys Ala
                245                 250                 255

ctc cca gcc ccc atc gag aaa acc atc tcc aaa gcc aaa ggg cag ccc 816
Leu Pro Ala Pro Ile Glu Lys Thr Ile Ser Lys Ala Lys Gly Gln Pro
            260                 265                 270

cga gaa cca cag gtg tac acc ctg ccc cca tcc cgg gat gag ctg acc 864
Arg Glu Pro Gln Val Tyr Thr Leu Pro Pro Ser Arg Asp Glu Leu Thr
        275                 280                 285

aag aac cag gtc agc ctg acc tgc ctg gtc aaa ggc ttc tat ccc agc 912

Lys Asn Gln Val Ser Leu Thr Cys Leu Val Lys Gly Phe Tyr Pro Ser
    290                 295                 300

gac atc gcc gtg gag tgg gag agc aat ggg cag ccg gag aac aac tac      960
Asp Ile Ala Val Glu Trp Glu Ser Asn Gly Gln Pro Glu Asn Asn Tyr
305                 310                 315                 320

aag acc acg cct ccc gtg ctg gac tcc gac ggc tcc ttc ttc ctc tac      1008
Lys Thr Thr Pro Pro Val Leu Asp Ser Asp Gly Ser Phe Phe Leu Tyr
                325                 330                 335

agc aag ctc acc gtg gac aag agc agg tgg cag cag ggg aac gtc ttc      1056
Ser Lys Leu Thr Val Asp Lys Ser Arg Trp Gln Gln Gly Asn Val Phe
            340                 345                 350

tca tgc tcc gtg atg cat gag gct ctg cac aac cac tac acg cag aag      1104
Ser Cys Ser Val Met His Glu Ala Leu His Asn His Tyr Thr Gln Lys
        355                 360                 365

agc ctc tcc ctg tct ccg ggt aaa tga                                  1131
Ser Leu Ser Leu Ser Pro Gly Lys
    370                 375

<210>  38
<211>  376
<212>  PRT
<213>  Homo sapiens

<400>  38

Ser Ser Asn Thr Ala Tyr Met Gln Leu Ser Ser Leu Thr Ser Glu Asp
1               5                   10                  15

Ser Ala Val Tyr Tyr Cys Ala Arg Gly Gly Phe Leu Gly His Tyr Gly
            20                  25                  30

Phe Ala Tyr Trp Gly Gln Gly Thr Leu Val Thr Val Ser Ala Ala Ser
        35                  40                  45

Thr Lys Gly Pro Ser Val Phe Pro Leu Ala Pro Ser Ser Lys Ser Thr
    50                  55                  60

Ser Gly Gly Thr Ala Ala Leu Gly Cys Leu Val Lys Asp Tyr Phe Pro
65                  70                  75                  80

Glu Pro Val Thr Val Ser Trp Asn Ser Gly Ala Leu Thr Ser Gly Val
                85                  90                  95

His Thr Phe Pro Ala Val Leu Gln Ser Ser Gly Leu Tyr Ser Leu Ser
            100                 105                 110

Ser Val Val Thr Val Pro Ser Ser Ser Leu Gly Thr Gln Thr Tyr Ile
        115                 120                 125

Cys Asn Val Asn His Lys Pro Ser Asn Thr Lys Val Asp Lys Lys Val
    130                 135                 140

Glu Pro Lys Ser Cys Asp Lys Thr His Thr Cys Pro Pro Cys Pro Ala
145                 150                 155                 160

Pro Glu Leu Leu Gly Gly Pro Ser Val Phe Leu Phe Pro Pro Lys Pro
                165                 170                 175

Lys Asp Thr Leu Met Ile Ser Arg Thr Pro Glu Val Thr Cys Val Val

            180                 185                 190

Val Asp Val Ser His Glu Asp Pro Glu Val Lys Phe Asn Trp Tyr Val
        195                 200                 205

Asp Gly Val Glu Val His Asn Ala Lys Thr Lys Pro Arg Glu Glu Gln
    210                 215                 220

Tyr Asn Ser Thr Tyr Arg Val Val Ser Val Leu Thr Val Leu His Gln
225                 230                 235                 240

Asp Trp Leu Asn Gly Lys Glu Tyr Lys Cys Lys Val Ser Asn Lys Ala
                245                 250                 255

Leu Pro Ala Pro Ile Glu Lys Thr Ile Ser Lys Ala Lys Gly Gln Pro
            260                 265                 270

Arg Glu Pro Gln Val Tyr Thr Leu Pro Pro Ser Arg Asp Glu Leu Thr
        275                 280                 285

Lys Asn Gln Val Ser Leu Thr Cys Leu Val Lys Gly Phe Tyr Pro Ser
    290                 295                 300

Asp Ile Ala Val Glu Trp Glu Ser Asn Gly Gln Pro Glu Asn Asn Tyr
305                 310                 315                 320

Lys Thr Thr Pro Pro Val Leu Asp Ser Asp Gly Ser Phe Phe Leu Tyr
                325                 330                 335

Ser Lys Leu Thr Val Asp Lys Ser Arg Trp Gln Gln Gly Asn Val Phe
            340                 345                 350

Ser Cys Ser Val Met His Glu Ala Leu His Asn His Tyr Thr Gln Lys
        355                 360                 365

Ser Leu Ser Leu Ser Pro Gly Lys
    370                 375

<210>  39
<211>  708
<212>  DNA
<213>  Homo sapiens

<220>
<221>  CDS
<222>  (1)..(705)

<400>  39
atg aga ccg tct att cag ttc ctg ggg ctc ttg ttg ttc tgg ctt cat      48
Met Arg Pro Ser Ile Gln Phe Leu Gly Leu Leu Leu Phe Trp Leu His
1               5                   10                  15

ggt gct cag tgt gac att cag ctg acc cag tct cca acc acc atg gct      96
Gly Ala Gln Cys Asp Ile Gln Leu Thr Gln Ser Pro Thr Thr Met Ala
            20                  25                  30

gca tct ccc ggg gag aag atc act atc acc tgc agt gcc agc tca agt      144
Ala Ser Pro Gly Glu Lys Ile Thr Ile Thr Cys Ser Ala Ser Ser Ser
        35                  40                  45

ata agt tcc aat tac ttg cat tgg tat cag cag aag cca gga ttc tcc      192
Ile Ser Ser Asn Tyr Leu His Trp Tyr Gln Gln Lys Pro Gly Phe Ser

    50                  55                  60

cct aaa ctc ttg att tat agg aca tcc aat ctg gct tct gga gtc cca 240
Pro Lys Leu Leu Ile Tyr Arg Thr Ser Asn Leu Ala Ser Gly Val Pro
65                  70                  75                  80

gct cgc ttc agt ggc agt ggg tct ggg acc tct tac tct ctc aca att 288
Ala Arg Phe Ser Gly Ser Gly Ser Gly Thr Ser Tyr Ser Leu Thr Ile
                85                  90                  95

ggc acc atg gag gct gaa gat gtt gcc act tac tac tgc cag cag ggt 336
Gly Thr Met Glu Ala Glu Asp Val Ala Thr Tyr Tyr Cys Gln Gln Gly
            100                 105                 110

agt agt ata ccg tac acg ttc gga ggg ggg acc aag ctg gag atc aaa 384
Ser Ser Ile Pro Tyr Thr Phe Gly Gly Gly Thr Lys Leu Glu Ile Lys
        115                 120                 125

cgt acg gtg gct gca cca tct gtc ttc atc ttc ccg cca tct gat gag 432
Arg Thr Val Ala Ala Pro Ser Val Phe Ile Phe Pro Pro Ser Asp Glu
    130                 135                 140

cag ttg aaa tct gga act gcc tct gtt gtg tgc ctg ctg aat aac ttc 480
Gln Leu Lys Ser Gly Thr Ala Ser Val Val Cys Leu Leu Asn Asn Phe
145                 150                 155                 160

tat ccc aga gag gcc aaa gta cag tgg aag gtg gat aac gcc ctc caa 528
Tyr Pro Arg Glu Ala Lys Val Gln Trp Lys Val Asp Asn Ala Leu Gln
                165                 170                 175

tcg ggt aac tcc cag gag agt gtc aca gag cag gac agc aag gac agc 576
Ser Gly Asn Ser Gln Glu Ser Val Thr Glu Gln Asp Ser Lys Asp Ser
            180                 185                 190

acc tac agc ctc agc agc acc ctg acg ctg agc aaa gca gac tac gag 624
Thr Tyr Ser Leu Ser Ser Thr Leu Thr Leu Ser Lys Ala Asp Tyr Glu
        195                 200                 205

aaa cac aaa gtc tac gcc tgc gaa gtc acc cat cag ggc ctg agc tcg 672
Lys His Lys Val Tyr Ala Cys Glu Val Thr His Gln Gly Leu Ser Ser
    210                 215                 220

ccc gtc aca aag agc ttc aac agg gga gag tgt tag 708
Pro Val Thr Lys Ser Phe Asn Arg Gly Glu Cys
225                 230                 235

<210> 40
<211> 235
<212> PRT
<213> Homo sapiens

<400> 40

Met Arg Pro Ser Ile Gln Phe Leu Gly Leu Leu Leu Phe Trp Leu His
1               5                   10                  15

Gly Ala Gln Cys Asp Ile Gln Leu Thr Gln Ser Pro Thr Thr Met Ala
            20                  25                  30

Ala Ser Pro Gly Glu Lys Ile Thr Ile Thr Cys Ser Ala Ser Ser Ser
        35                  40                  45

Ile Ser Ser Asn Tyr Leu His Trp Tyr Gln Gln Lys Pro Gly Phe Ser
    50                  55                  60

Pro Lys Leu Leu Ile Tyr Arg Thr Ser Asn Leu Ala Ser Gly Val Pro
65                  70                  75                  80

Ala Arg Phe Ser Gly Ser Gly Ser Gly Thr Ser Tyr Ser Leu Thr Ile
                85                  90                  95

Gly Thr Met Glu Ala Glu Asp Val Ala Thr Tyr Tyr Cys Gln Gln Gly
            100                 105                 110

Ser Ser Ile Pro Tyr Thr Phe Gly Gly Gly Thr Lys Leu Glu Ile Lys
        115                 120                 125

Arg Thr Val Ala Ala Pro Ser Val Phe Ile Phe Pro Pro Ser Asp Glu
    130                 135                 140

Gln Leu Lys Ser Gly Thr Ala Ser Val Val Cys Leu Leu Asn Asn Phe
145                 150                 155                 160

Tyr Pro Arg Glu Ala Lys Val Gln Trp Lys Val Asp Asn Ala Leu Gln
                165                 170                 175

Ser Gly Asn Ser Gln Glu Ser Val Thr Glu Gln Asp Ser Lys Asp Ser
            180                 185                 190

Thr Tyr Ser Leu Ser Ser Thr Leu Thr Leu Ser Lys Ala Asp Tyr Glu
        195                 200                 205

Lys His Lys Val Tyr Ala Cys Glu Val Thr His Gln Gly Leu Ser Ser
    210                 215                 220

Pro Val Thr Lys Ser Phe Asn Arg Gly Glu Cys
225                 230                 235

<210> 41
<211> 21
<212> DNA
<213> Artificial

<220>
<223> Synthetic DNA

<400> 41
gctgcagtgt ctgaaagatt t 21

<210> 42
<211> 124
<212> DNA
<213> Artificial

<220>
<223> Synthetic DNA

<400> 42
gtttgctgca gtgtttgaaa gatttgtgtg ctgtccaaat ctttcagaca ctgcagcttt 60

ttcgacgtca caaactttct aaacacacga caggtttaga aagtctgtga cgtcgaaaaa 120

cgta 124

<210> 43
<211> 62
<212> DNA
<213> Artificial

<220>
<223> Synthetic DNA

<400> 43
atgcaaaaag ctgcagtgtc tgaaagattt ggacagcaca caaatctttc aaacactgca 60

gc                                                                    62

<210>  44
<211>  19
<212>  DNA
<213>  Artificial

<220>
<223>  Synthetic DNA

<400>  44
gcgtttctca taccgtatt                                                  19

<210>  45
<211>  116
<212>  DNA
<213>  Artificial

<220>
<223>  Synthetic DNA

<400>  45
gtttgcgttt ctcatactgt attgtgtgct gtccaatacg gtatgagaaa cgctttttcg      60

caaagagtat gacataacac acgacaggtt atgccatact ctttgcgaaa aacgta          116

<210>  46
<211>  58
<212>  DNA
<213>  Artificial

<220>
<223>  Synthetic DNA

<400> 46
atgcaaaaag cgtttctcat accgtattgg acagcacaca atacagtatg agaaacgc 58

<210> 47
<211> 21
<212> DNA
<213> Artificial

<220>
<223> Synthetic DNA

<400> 47
gctatgcctc cgttgtaatt g 21

<210> 48
<211> 124
<212> DNA
<213> Artificial

<220>
<223> Synthetic DNA

<400> 48
gtttgctatg cctctgttgt aattggtgtg ctgtcccaat tacaacggag gcatagcttt 60

ttcgatacgg agacaacatt aaccacacga cagggttaat gttgcctccg tatcgaaaaa 120

cgta 124

<210> 49
<211> 62
<212> DNA
<213> Artificial

<220>

<223>  Synthetic DNA

<400>  49
atgcaaaaag ctatgcctcc gttgtaattg ggacagcaca ccaattacaa cagaggcata      60

gc                                                                      62

<210>  50
<211>  21
<212>  DNA
<213>  Artificial

<220>
<223>  Synthetic DNA

<400>  50
gacctacaac aaaacatacg t                                                 21

<210>  51
<211>  124
<212>  DNA
<213>  Artificial

<220>
<223>  Synthetic DNA

<400>  51
gtttgaccta caacaaaata tacgtgtgtg ctgtccacgt atgttttgtt gtaggtcttt      60

ttctggatgt tgttttatat gcacacacga caggtgcata caaaacaaca tccagaaaaa     120

cgta                                                                   124

<210>  52
<211>  62

<212> DNA
<213> Artificial

<220>
<223> Synthetic DNA

<400> 52
atgcaaaaag acctacaaca aaacatacgt ggacagcaca cacgtatatt ttgttgtagg 60

tc 62

## Description

### TECHNICAL FIELD

The present invention relates to a composition for treatment of SLC6A6-expressing cancer cells. More specifically, the present invention relates to a novel composition for cancer treatment using a monoclonal antibody or an antibody fragment, which binds to SLC6A6 or its extracellular region, or a substance which suppresses SLC6A6 expression.

### BACKGROUND ART

Cancer ranks high in the causes of death in the world. Above all, colorectal cancer is a disease being at a higher position in the mortality of cancer. In Japan, the number of colorectal cancer patients has been suddenly increasing in recent years, and about 60,000 patients suffer from colorectal cancer every year. In the number of deaths classified by organ system, colorectal cancer ranks third after gastric cancer and lung cancer. Colorectal cancer has a five-year survival rate of about 90% or more when remaining only in the colon or rectum, and is therefore known as a cancer for which early diagnosis leads to a higher healing rate. In spite of this fact, colorectal cancer is a high-mortality cancer. This is because colorectal cancer not only has high morbidity, but also shows a sudden increase in mortality with the progression of cancer, i.e., its five-year survival rate is reduced to 70% upon metastasis to lymph nodes and reduced to 25% or less upon distant metastasis to lung or liver. For treatment of such colorectal cancer, surgical treatment and chemotherapy are commonly used, while attempts have also been made to search for cancer-specific new therapies since the recent appearance of molecular targeted drugs. Among molecular targeted cancer therapeutic agents, antibody drugs such as Herceptin for breast cancer and Rituxan for non-Hodgkin's lymphoma exert high therapeutic effects. These antibody drugs are known to exert their efficacy through binding to proteins (Her2 and CD20, respectively) present on the cell membrane.

As antibody drugs approved in Japan for use as molecular targeted drugs for colorectal cancer, Avastin and Erbitux are known. They are antibody drugs targeted at growth factors such as VEGF and EGF. Avastin is approved for use in progressive and recurrent colorectal cancer for which curative resection is impossible. Avastin works by a mechanism of action which involves binding to VEGF to prevent its binding to VEGF receptors, thereby inhibiting vascularization and blocking nutrition to tumor tissues.

Erbitux is intended to stop the proliferation of cancer cells through binding to

EGF receptors and thereby inhibiting EGF-mediated cell proliferation signals. Moreover, another mechanism of action also appears to work, i.e., antibody biding to the surface of cancer cells will cause antibody-dependent cellular cytotoxicity (ADCC) mediated by natural killer cells (NK cells) and/or macrophages, etc., whereby the cancer cells will be killed.

Further, other mechanisms required for antibody drugs to exert their efficacy include the EPR (enhanced permeability and retention) effect, i.e., tumor accumulation of antibody molecules. The vascular permeability is significantly enhanced in tumor tissues when compared to normal tissues, so that tumor tissues are more likely to cause leakage of macromolecules and/or microparticles from blood vessels, while substances which have reached tumor tissues are accumulated therein because the lymphatic system is not developed in tumor tissues. Since tumor tissues have such properties, it is known that 40 kDa or larger molecules including antibodies are more likely to accumulate in tumors and more likely to exert their efficacy, which is called the EPR effect (Non-patent Document 1). This effect serves as a base for the development of drug delivery for effective drug transport to tumors.

In addition to antibody drugs, recent efforts have also been made to develop therapeutic agents based on RNA interference which involves introduction of double-stranded RNA into cells to disrupt a target gene (mRNA) and thereby suppress its expression. RNA interference, which is one of the techniques for cancer treatment by suppressing the expression of a target involved in the proliferation and/or metastasis of cancer cells, is now being developed toward practical application.

On the other hand, molecular targeted drugs will also affect normal cells and hence may cause lethal side effects in some cases. For example, Herceptin, which is a therapeutic agent for breast cancer, may cause not only headache, asthenia, nausea and vomiting, but also interstitial pneumonia, bone marrow inhibition, hepatic disorders, renal disorders and cerebrovascular disorders. Moreover, in tissue staining, Herceptin is also known to strongly react with normal cardiomyocytes to thereby cause severe cardiac disorders (Non-patent Document 2). Further, Herceptin is an antibody drug targeted at Her2 and hence is effective only for patients who express Her2.

In the case of Avastin, which is a therapeutic agent for colorectal cancer, its side effects include hemorrhage, thrombosis, gastrointestinal perforation, delayed wound healing, increased blood pressure and so on, among which thrombosis and gastrointestinal perforation are fatal side effects (Non-patent Document 3). Side effects known for Erbitux include skin disorders and so on, which are not fatal but cause itching and white pustules, resulting in mental and physical burdens on patients (Non-patent Document 4). Moreover, Erbitux also has a problem in that it has no effect on canceration caused by a change in signals downstream of EGFR (e.g., K-ras mutation).

In view of the foregoing, antibody drugs against cancers still have problems, e.g., in that they will cause severe side effects and are effective only for limited patients. Thus, there has been a demand for new development of cancer-specific molecular targets and pharmaceutical preparations with fewer side effects.

Under these circumstances, the inventors of the present invention have narrowed down genes whose expression is enhanced specifically in colorectal cancer cells and have focused on SLC6A6 (solute carrier family 6 (neurotransmitter transporter, taurine), member 6) as a membrane protein molecule expressed specifically in cancer cells.

SLC6A6 is a 12-transmembrane protein consisting of 620 amino acids and has been registered at NCBI (the National Center for Biotechnology Information) under Reference Sequences [RefSeq] ID: NM_003043 and NP_003034.2 (SEQ ID NO: 1: nucleotide sequence, SEQ ID NO: 2: amino acid sequence). SLC6A6 is involved in taurine uptake into cells and transports taurine together with sodium ions and chloride ions.

The SLC6A6 gene has been disclosed as one of the genes whose expression is increased in colon cancer tissue when compared to normal tissue (Patent Document 1). In Patent Document 1, 30 or more genes including slc6a6, whose expression differs between colon cancer tissue and normal tissue, have been identified by DNA microarray analysis using 10 primary colon tumors and 10 normal colon samples, and the disclosed genes are all suggested to be applied to antibody design, antibody-mediated polypeptide detection, cancer diagnosis, and antibody-containing pharmaceutical compositions. However, there is no preparation example of an antibody against slc6a6, and there is also no positive evidence for its adaptability, practicality or enablement at the protein level for diagnostic or therapeutic purposes. Moreover, Patent Document 1 fails to verify the involvement of SLC6A6 protein in the proliferation and/or metastasis of cancer cells. Further, Patent Document 1 shows the detailed analysis results of expression patterns by quantitative PCR assay, but there are variations in the expression ratio and some cases show higher expression in normal tissue than in colon cancer tissue (Table 7). In addition, the primers used for quantitative PCR (Table 6) are located outside the protein coding regions. For use as a therapeutic agent, an antibody which detects a protein is required, while an antibody which binds to the cell membrane region is also required to ensure antibody binding to living cancer cells. For use as an antibody drug, a monoclonal antibody which recognizes a specific amino acid sequence and a three-dimensional structure is indispensable and should be studied in detail for its usefulness as a therapeutic agent. Moreover, for development of RNA interference-mediated pharmaceutical preparations, a sequence capable of suppressing SLC6A6 expression should be clarified and used to suppress SLC6A6 expression, whereby the usefulness of each pharmaceutical preparation should be studied in detail for its effects on the proliferation and/or metastasis of cancer cells.

As antibodies against SLC6A6, a plurality of antibodies are known, including HPA015028 (ATLAS) and sc-166640 (SantaCruz). For development as antibody drugs, these antibodies should be monoclonal because they are required to be chimerized or humanized. HPA015028 is an antibody recognizing the extracellular region comprising amino acid residues 143 to 216 (SEQ ID NO: 3 (nucleotide sequence), SEQ ID NO: 4 (amino acid sequence)), but it is a polyclonal antibody. Among known antibodies, sc-166640 is the only monoclonal antibody. However, this antibody binds to a region covering amino acid residues 397 to 424 of SLC6A6. This region extends from the transmembrane domain to the intracellular domain, and hence this antibody cannot bind to living cancer cells and cannot be used as a therapeutic agent.

For the foregoing reasons, these conventional antibodies are not sufficient as antibodies which can be used as antibody drugs. Moreover, there has been no analysis as to what effect occurs on cancer cells upon suppression of SLC6A6 expression.
Patent Document 1: JP 2006-515318 A
Non-patent Document 1: Cancer Research, 44, 2115-2121, 1984
Non-patent Document 2: British Journal of Cancer, 94, 1016-1020, 2006
Non-patent Document 3: Cancer Research, 57, 4593-4599, 1997
Non-patent Document 4: Journal of Clinical Oncology, 22, 1201-1208, 2004

### DISCLOSURE OF THE INVENTION

In general, surgical treatment of cancer has problems not only in that it is difficult to treat metastatic lesions, but also in that it involves invasion and occurrence of complications. Moreover, chemotherapy and radiation therapy have problems of side effects. Further, conventional antibody drugs not only cause side effects, but also have no effect on some cancers. For these reasons, there has been a demand for the development of new pharmaceutical preparations against cancers.

The present invention is based on the finding that the membrane protein SLC6A6 is a protein overexpressed in cancer tissues and is a useful marker as a target of diagnosis and treatment. The present invention aims to provide an anticancer agent comprising, as an active ingredient, a monoclonal antibody or a fragment thereof, which binds to SLC6A6 expressed specifically in colorectal cancer and can be stably supplied as a cancer therapeutic agent. The present invention also aims to provide an anticancer agent comprising, as an active ingredient, a substance capable of suppressing SLC6A6 expression, i.e., siRNA, shRNA or an expression vector allowing their expression.

As a result of repeating extensive and intensive efforts to solve the problems stated above, the inventors of the present invention have prepared a monoclonal antibody recognizing amino acid residues 143 to 216 in the extracellular region of SLC6A6 and have succeeded in developing an antibody for cancer treatment comprising such a monoclonal antibody. This has led to the completion of the present invention. Moreover, with the use of cells overexpressing SLC6A6, the inventors of the present invention have identified a plurality of nucleic acid sequences for RNAi, which are capable of suppressing SLC6A6 expression. This has led to the completion of the present invention. Namely, the present invention is as follows.
(1) A pharmaceutical composition comprising a monoclonal antibody or a fragment thereof, which recognizes native SLC6A6, or a substance capable of suppressing SLC6A6 expression by RNA interference.
(2) A pharmaceutical composition comprising a monoclonal antibody or a fragment thereof, which recognizes a polypeptide in the extracellular region of SLC6A6.
(3) The pharmaceutical composition according to (2) above, wherein the polypeptide in the extracellular region is at least one selected from (a) to (c) shown below:
   (a) a polypeptide which consists of the amino acid sequence shown in SEQ ID NO:4;
   (b) a polypeptide which consists of an amino acid sequence with substitution, deletion and/or insertion of one or several amino acids in the amino acid sequence shown in SEQ ID NO: 4 and which serves as an extracellular region of SLC6A6; and
   (c) a polypeptide which consists of an amino acid sequence sharing a homology of 70% or more with the amino acid sequence shown in SEQ ID NO: 4 and which serves as an extracellular region of SLC6A6.
(4) A pharmaceutical composition comprising a monoclonal antibody against SLC6A6 or a fragment thereof, which is produced by a hybridoma having Accession No. FERM BP-11413 or FERM BP-11414.
(5) A pharmaceutical composition comprising a monoclonal antibody against SLC6A6 or a fragment thereof, which binds to an epitope recognized by the monoclonal antibody or fragment thereof according to (4) above.
(6) The pharmaceutical composition according to any one of (1) to (5) above, wherein the monoclonal antibody is human or humanized.
(7) The pharmaceutical composition according to any one of (1) to (5) above, wherein the monoclonal antibody is fused.
(8) The pharmaceutical composition according to any one of (1) to (7) above, which is intended for treatment of colorectal cancer.

The present invention provides a pharmaceutical composition, particularly a pharmaceutical composition for colorectal cancer treatment, which comprises a humanized, chimeric or human anti-SLC6A6 monoclonal antibody or a fragment thereof, or an antibody fusion protein or a fragment thereof. The antibody, fusion protein and fragments thereof in the present invention may be used alone or by being bound to at least one therapeutic agent or in combination with another mode of therapy. Moreover, a substance capable of suppressing SLC6A6 expression can be used as a therapeutic agent by being transported to a target organ through an existing drug delivery system to thereby suppress the proliferation, invasion and migration of cancer cells.

### BRIEF DESCRIPTION OF THE DRAWINGS

Figure 1 shows photographs of tissues stained by *in situ* hybridization using a nucleic acid having a nucleotide sequence corresponding to the extracellular region of SLC6A6 protein as a probe.
Figure 2 shows the analysis results of SLC6A6-expressing cells.
Figure 3 shows the results of ELISA analysis obtained for monoclonal antibodies reacting with a peptide of amino acid residues 145 to 213 in the extracellular region of SLC6A6.
Figure 4 shows colorectal cancer tissues stained with an anti-SLC6A6 antibody, 4B9b. Only cancer lesions were stained specifically.
Figure 5 shows normal tissues stained with an anti-SLC6A6 antibody, 4B9b. Only colorectal cancer was stained specifically.
Figure 6 shows the results of SLC6A6 expression in 10 lines of colorectal cancer cells, as analyzed by real-time quantitative RT-PCR. SLC6A6 expression was detected in all lines although they showed different expression levels.
Figure 7 shows the results obtained for 10 lines of colorectal cancer cells, as analyzed with 4B9b antibody and 5H12d antibody. All lines of colorectal cancer cells were found to react with these antibodies.
Figure 8 shows the results of ELISA analysis on the activity of a mouse IgG-chimerized antibody.
Figure 9 shows cancer tissues and normal tissues stained with a mouse IgG-chimerized antibody, 4B9b. It is indicated that stronger staining was observed at the invasion front where cancer cells have invaded.
Figure 10 shows the results of ELISA analysis on the activity of a human IgG-chimerized antibody.
Figure 11 shows the results of FACS analysis obtained for subclass IgG antibodies. 19B10, 7C11 and 12E8 were found to recognize SLC6A6 on the cell membrane.
Figure 12 shows the cell morphology of mouse breast cancer 4T1 cells where SLC6A6 is overexpressed.
Figure 13 shows the results observed for changes in E-cadherin, N-cadherin and vimentin in SLC6A6-overexpressing cells. β-Actin was used for the purpose of confirming differences in the protein amounts used in electrophoresis. A change in phenotype as seen in EMT was observed.
Figure 14 shows the results observed for increases in tumor size upon antibody addition in Balb/c mice transplanted with SLC6A6-overexpressing cell lines (L31 and L35) and a non-expressing cell line (4T1). It was indicated that antibody-induced suppression of proliferation was observed only in the expressing cell lines.

### BEST MODES FOR CARRYING OUT THE INVENTION

The present invention will be described in more detail below. It should be noted that the present invention is not limited to the following embodiments and can be implemented with modifications as appropriate within the spirit of the present invention.

It should be noted that all publications cited herein, including prior art documents, patent gazettes and other patent documents, are incorporated herein by reference. Moreover, this specification incorporates the contents disclosed in the specification and drawings of Japanese Patent Application No. 2012-049192 (filed on March 6, 2012), based on which the present application claims priority.

The present invention relates to a pharmaceutical composition comprising a monoclonal antibody which recognizes a molecule called SLC6A6 (solute carrier family 6 (neurotransmitter transporter, taurine), member 6) or its extracellular region. In particular, this antibody specifically binds to colorectal cancer cells. Moreover, the present invention uses a vector comprising shRNA or siRNA which suppresses SLC6A6 expression. This shRNA or siRNA specifically suppresses the proliferation and metastasis of colorectal cancer cells. Thus, the pharmaceutical composition of the present invention is useful for treatment of cancer, particularly colorectal cancer.

To obtain the monoclonal antibody of the present invention, human SLC6A6 in a state of retaining its original three-dimensional structure, i.e., its full-length protein (SEQ ID NO: 1 (nucleotide sequence), SEQ ID NO: 2 (amino acid sequence)) or a partial protein thereof comprising an extracellular region, i.e., amino acid residues 143 to 216 (SEQ ID NO: 3 (nucleotide sequence), SEQ ID NO: 4 (amino acid sequence)) (which are also hereinafter collectively referred to as a protein) is used as an immunogen. For recognition of living cells, an antibody capable of recognizing the original three-dimensional structure of a membrane protein is obtained.

The monoclonal antibody to be used in the pharmaceutical composition of the present invention (hereinafter also referred to as "the monoclonal antibody of the present invention") is capable of recognizing native SLC6A6. The term "native" is intended to mean being in a state of retaining the three-dimensional structure which is taken by the intended protein in an *in vivo* environment.

Moreover, the monoclonal antibody of the present invention is capable of recognizing the extracellular region of SLC6A6. In particular, it is capable of recognizing a region covering amino acid residues 143 to 216 of SLC6A6 (SEQ ID NO: 4) as an extracellular region.

Within the range of retaining the binding activity to a polypeptide having the amino acid sequence shown in SEQ ID NO: 4, i.e., as long as a target polypeptide has functions as the extracellular region of SLC6A6, the monoclonal antibody of the present invention may also recognize a mutated polypeptide comprising substitution, deletion or insertion of one or several (e.g., 2 to 20, preferably 2 to 10, more preferably 2, 3, 4 or 5) amino acids in the amino acid sequence shown in SEQ ID NO: 4 or a mutated polypeptide sharing a homology of 70% or more, preferably 80% or more, 90% or more, 95% or more, or 98% or more with the amino acid sequence shown in SEQ ID NO: 4.

The extracellular region of SLC6A6 is predicted to be responsible for binding with taurine and transport of taurine into cells. Confirmation of whether or not a mutated polypeptide has functions as the extracellular domain of SLC6A6 would be able to be accomplished by forcing the mutated polypeptide to be expressed in animal cells or the like and analyzing taurine uptake by the activation method (J. Membr. Biol, 76, 1-15, 1983).

Alternatively, since the monoclonal antibody of the present invention binds to SLC6A6, among the above mutated polypeptides, those to which the monoclonal antibody of the present invention can bind indicate that the antibody maintains its binding activity to a polypeptide having the amino acid sequence shown in SEQ ID NO: 3, i.e., they fall within polypeptides having functions as the extracellular region of SLC6A6.

Binding between a mutated polypeptide and the monoclonal antibody of the present invention may be confirmed by ELISA, immunoprecipitation, western blotting, etc.

Moreover, the extracellular region of SLC6A6 corresponds to a cell surface site of a marker protein whose expression is increased in cancer cells. Confirmation of whether or not a mutated polypeptide has functions as the extracellular domain of SLC6A6 may be accomplished by comparing the expression of the mutated polypeptide between normal cells and cancer cells by means of immunostaining, ELISA, immunoprecipitation, western blotting, FACS, etc.

The monoclonal antibody of the present invention has higher affinity against SLC6A6 than conventional antibodies.

Conventional antibodies have problems in that they cannot bind to living cells because of having no ability to recognize an extracellular region, and hence cannot be used as therapeutic agents. In contrast, the monoclonal antibody of the present invention not only recognizes an extracellular region, unlike conventional antibodies, but also has higher affinity than conventional antibodies; and hence it binds with high affinity to SLC6A6 present on the cancer cell surface to thereby directly suppress the functions of SLC6A6 *in vivo* and also can lead cancer cells to death through ADCC (antibody-dependent cellular cytotoxicity) activity.

The monoclonal antibody of the present invention also recognizes proteins encoded by mRNA variants of slc6a6. The monoclonal antibody of the present invention can bind not only to full-length SLC6A6, but also to partially deficient mutants thereof, and is therefore capable of binding to a wide range of SLC6A6-expressing cancer cells.

In the present invention, various genetic engineering and protein engineering procedures can be used to prepare antibody fragments which are portions of the monoclonal antibody, antibody-like molecules (e.g., low molecular antibody, genetically recombinant antibody, modified antibody), or a protein fused with the monoclonal antibody. More specifically, examples include H chain, L chain, Fv, Fab, Fab', F(ab')2, scFv, sdFv, sc(Fv)2, (scFv)2, DiAbody, chimeric antibody, humanized antibody, human antibody, single chain antibody, multi-specific antibody (e.g., bispecific antibody), labeled antibody and so on. All of them fall within the monoclonal antibody of the present invention as long as they are molecules having the ability to bind to the extracellular region of SLC6A6.

Cell lines (hybridomas) producing the monoclonal antibody of the present invention are designated as "Mouse-Mouse hybridoma 4B9b" (hereinafter referred to as "4B9b") and "mouse-mouse hybridoma 5H12d" (hereinafter referred to as "5H12d"), both of which were deposited on July 21, 2010 with the International Patent Organism Depositary, the National Institute of Advanced Industrial Science and Technology (Central 6, 1-1-1 Higashi, Tsukuba-shi, Ibaraki 305-8566, Japan) by Bio Matrix Research Inc. (105 Higashifukai, Nagareyama-shi, Chiba 270-0101, Japan). Accession No. is "FERM BP-11413" for 4B9b and "FERM BP-11414" for 5H12d. In addition, the following cell lines were further obtained: "3A3," "19B10," "23G12," "7C11," "12E8," "6G3," "18A10," "22A4," "23H6," "26F3," "2F2," "7H8" and "19C2j." "3A3," "19B10," "23G12," "7C11" and "12E8" were found to produce subclass IgG antibodies, while the others were found to produce IgM antibodies. The present invention provides these hybridomas and antibodies produced therefrom. When these hybridomas are cultured, it is possible to prepare homogeneous monoclonal antibodies.

The monoclonal antibody of the present invention having these features is obtained by immunization procedures where cells engineered to express a membrane protein are allowed to be engrafted, as disclosed in WO/2010/098471, unlike commonly used procedures for monoclonal antibody preparation. The monoclonal antibody of the present invention is difficult to prepare by procedures commonly used by those skilled in the art. This is because:
(1) when a surfactant is used to prepare a membrane protein for use as an antigen, the membrane protein will lose its three-dimensional structure, whereas when no surfactant is used, aggregation will occur between hydrophobic regions in the membrane protein; and
(2) no immune response is induced because the expression level on the cell surface is low or because the extracellular region is small.

The monoclonal antibody of the present invention has acquired advantageous features over conventional antibodies as a result of inventive modifications made by the inventors of the present invention to procedures for antibody preparation (particularly immunization procedures).

The monoclonal antibody of the present invention has the following features.

The antibody of the present invention can recognize native SLC6A6 because it is prepared based on the three-dimensional structure originally possessed by SLC6A6. For this reason, the antibody of the present invention has very strong binding ability in comparison with a conventional antibody (HPA015028) which recognizes the same epitope, and hence the antibody of the present invention achieves sufficient binding to SLC6A6 on the cell membrane, which has been difficult with conventional antibodies. Moreover, unlike a conventional monoclonal antibody (sc-166640) which recognizes the intramembrane and intracellular regions of SLC6A6, the antibody of the present invention has a recognition site in the extracellular region, and is therefore capable of binding to living cells and can be used as a therapeutic agent.

In addition, conventional antibodies are polyclonal antibodies and have been difficult to produce continuously as homogeneous antibodies, whereas the antibody of the present invention is a monoclonal antibody and hence can be mass-produced with high reproducibility. In terms of these features, the antibody of the present invention will be able to reduce the cancer-related mortality when used for cancer treatment.

Moreover, the present invention is not limited only to the monoclonal antibody against SLC6A6, which is produced from a hybridoma having Accession No. FERM BP-11413 or FERM BP-11414, and any other antibodies also fall within the monoclonal antibody against SLC6A6 in the present invention as long as they bind to an epitope which is recognized by monoclonal antibodies produced from these hybridomas. As used herein, the term "epitope" refers to an epitope which is recognized by monoclonal antibodies produced from the above hybridomas (i.e., amino acid residues 145 to 213 in the amino acid sequence of SLC6A6, or a partial region thereof).

The antibody against SLC6A6 may be a humanized antibody or a human antibody. A humanized antibody, e.g., a mouse-human chimeric antibody, may be prepared by isolating antibody genes from mouse cells producing an antibody against SLC6A6 protein and causing recombination between its H chain constant region and human IgG H chain constant region gene, followed by introduction into mouse myeloma cells. On the other hand, a human antibody may be prepared by immunizing the SLC6A6 protein into mice whose immune system has been replaced with the human immune system, as disclosed in WO/2010/098471. A protein fused with a monoclonal antibody may be prepared using the antigen-binding variable region of the antibody and another protein by existing procedures for gene recombination. Alternatively, it may be prepared by crosslinking the monoclonal antibody and the protein through a crosslinker.

The cancer therapeutic agent of the present invention may optionally comprise a pharmaceutically acceptable anticancer agent or carrier, as appropriate, in addition to the anti-SLC6A6 antibody.

In the present invention, SLC6A6 expression is suppressed to prevent the proliferation and metastasis of cancer cells, whereby cancer progression and metastasis can be suppressed. Namely, it is possible to provide a substance which suppresses SLC6A6 expression by the technique called RNA interference (RNAi). When using siRNA (small interfering RNA) or shRNA (short hairpin RNA) or an expression vector allowing their expression as an active ingredient, such a substance may be used as a cancer therapeutic agent (i.e., an inhibitor of cancer progression or metastasis) through an existing drug delivery system.

Specific examples of a substance capable of suppressing SLC6A6 expression by RNAi include siRNA, shRNA, or expression vectors allowing their expression, and so on. When these substances are introduced into cells, RNAi phenomenon will occur to thereby cause degradation of RNA having homologous sequences. Such RNAi phenomenon is observed in nematodes, insects, protozoan, hydras, plants and vertebrate animals (including mammals).

In the present invention, it is possible to use double-stranded RNA called siRNA having a length of about 20 bases (e.g., about 21 to 23 bases) or less. Such siRNA suppresses gene expression when expressed in cells, so that a gene targeted by the siRNA (the SLC6A6 gene in the present invention) can be prevented from being expressed.

siRNA used in the present invention may be of any form as long as it can cause RNAi. As used herein, the term "siRNA" is an abbreviation of short interfering RNA and refers to short double-stranded RNA of 10 base pairs or more, which is artificially synthesized either chemically or biochemically, or synthesized *in vivo,* or generated upon *in vivo* degradation of double-stranded RNA of about 40 bases or more. siRNA generally has a structure with 5'-phosphate and 3'-OH and has an overhang of about 2 bases at the 3'-terminal end. A specific protein binds to this siRNA to form RISC (RNA-induced silencing complex). This complex recognizes and binds to mRNA having the same sequence as the siRNA and thereby cleaves the mRNA at the center of the siRNA by RNaseIII-like enzyme activity. It is preferred that the sequence of the siRNA is 100% identical with the sequence of the mRNA to be cleaved as a target. However, even when bases located at positions outside the center of the siRNA are not identical, RNAi-mediated cleavage activity often remains partially intact, so that 100% identity is not always required.

It is preferred that a region homologous between the nucleotide sequence of siRNA and the nucleotide sequence of the SLC6A6 gene whose expression is to be suppressed does not comprise the translation initiation region of the SLC6A6 gene. This is because various transcription factors and translation factors are predicted to bind to the translation initiation region, which prevents siRNA from effectively binding to mRNA and hence results in a reduction in the effect. Thus, a homologous sequence is preferably 20 bases apart from the translation initiation region of the SLC6A6 gene, and more preferably 70 bases apart from the translation initiation region of the SLC6A6 gene. Such a homologous sequence may be, for example, a sequence near the 3'-terminal end of the SLC6A6 gene. Further, when transcribed as mRNA, a sequence may be prepared in a region (3'-UTR) of mRNA downstream of the stop codon for the protein-coding region in the SLC6A6 gene.

In the present invention, siRNA can be used as a factor for causing RNAi, and a factor responsible for siRNA production (e.g., dsRNA of about 40 bases or more) may be used as such a factor. For example, it is possible to use double-stranded RNA or a variant thereof, which comprises a sequence sharing a homology of at least about 70%, preferably 75% or more, more preferably 80% or more, even more preferably 85% or more, still even more preferably 90% or more, particularly preferably 95% or more, most preferably 100% with a part of the nucleic acid sequence of the SLC6A6 gene (SEQ ID NO: 1 or 3). Such a homologous sequence segment usually has a length of at least about 15 nucleotides or more, preferably at least about 19 nucleotides, more preferably at least about 20 nucleotides, and even more preferably at least about 21 nucleotides.

According to another embodiment of the present invention, shRNA (short hairpin RNA) consisting of a short hairpin structure with an overhang at the 3'-terminal end can be used as a factor capable of suppressing SLC6A6 expression by RNAi. shRNA refers to a single-stranded RNA molecule of about 20 base pairs or more, which has a hairpin-like structure as a result of comprising a partially palindromic nucleotide sequence and thereby forming a double-stranded structure within the molecule. Upon introduction into cells, such shRNA is degraded within the cells into segments of about 20 bases (typically, e.g., 21 bases, 22 bases, 23 bases) in length, as a result of which RNAi can be caused as in the case of siRNA. As described above, shRNA causes RNAi as in the case of siRNA and therefore can be used effectively in the present invention.

shRNA preferably has a 3'-overhang end. The double-stranded segment may be of any length, preferably about 10 nucleotides or more, and more preferably about 20 nucleotides or more. In this case, the 3'-overhang end is preferably DNA, more preferably DNA of at least 2 nucleotides or more, and even more preferably DNA of 2 to 4 nucleotides.

For use in the present invention, a substance capable of suppressing SLC6A6 expression by RNAi (i.e., siRNA or shRNA as described above) may be artificially prepared by chemical synthesis or may be prepared by *in vitro* synthesis with T7 RNA polymerase from hairpin structure DNA composed of sense and antisense DNA sequences linked in the reverse direction. In the case of *in vitro* synthesis, T7 RNA polymerase and T7 promoter may be used to synthesize antisense and sense RNAs from the template DNA. When these RNAs are annealed *in vitro* and then introduced into cells, RNAi will be caused to suppress SLC6A6 expression. In this case, such RNAs may be introduced into cells by calcium phosphate transfection or using various transfection reagents (e.g., oligofectamine, lipofectamine and lipofection), by way of example.

Further, in the present invention, it is possible to use an expression vector comprising a nucleic acid sequence encoding a substance capable of suppressing SLC6A6 expression by RNAi (preferably siRNA or shRNA).

The cancer therapeutic agent of the present invention can be used widely for cancer treatment, and preferably can be used as a pharmaceutical agent for suppressing carcinogenesis and cancer invasion and/or metastasis.

The cancer therapeutic agent of the present invention may be administered to any type of cancer where SLC6A6 is expressed, and examples include malignant melanoma, malignant lymphoma, digestive organ cancer, lung cancer, esophageal cancer, gastric cancer, colorectal cancer, rectal cancer, colon cancer, urinary tract tumor, gallbladder cancer, bile duct cancer, biliary tract cancer, breast cancer, liver cancer, pancreatic cancer, testicular tumor, maxillary cancer, tongue cancer, lip cancer, oral cancer, pharyngeal cancer, laryngeal cancer, kidney cancer, ovarian cancer, uterine cancer, prostate cancer, thyroid cancer, brain tumor, Kaposi's sarcoma, angioma, leukemia, polycythemia vera, neuroblastoma, retinoblastoma, myeloma, urinary bladder tumor, sarcoma, osteosarcoma, myosarcoma, skin cancer, basal cell carcinoma, skin appendage carcinoma, skin metastatic cancer, skin melanoma and so on. Preferred are colorectal cancer, gastric cancer, bladder cancer, kidney cancer, uterine cancer and breast cancer, and more preferred are colorectal cancer and uterine cancer.

The cancer therapeutic agent of the present invention may be administered in any mode, including parenteral administration (e.g., subcutaneous administration, intracutaneous administration, mucosal administration, intrarectal administration, intravaginal administration, topical administration to the affected area, dermal administration), direct administration to the affected area, etc.

The dose of the pharmaceutical composition of the present invention may generally be determined as appropriate for the age and body weight of a subject (patient) to be administered, the type and progression of disease, the route of administration, the frequency of administration, the period of administration, etc., in consideration of the mixing ratio of the active ingredient (the monoclonal antibody of the present invention or a substance capable of suppressing SLC6A6 expression by RNAi) in the formulation.

Detailed explanation will be given below for the case where the pharmaceutical composition of the present invention is used as a parenteral formulation.

For use as a parenteral formulation, the pharmaceutical composition of the present invention may usually be formulated into any dosage form, such as intravenous injections (including drip infusions), intramuscular injections, intraperitoneal injections, subcutaneous injections, suppositories, etc. In the case of various types of injections, for example, they may be provided in the form of unit dose ampules or multi-dose containers or as freeze-dried powders which are dissolved again in a diluent before use. Such a parenteral formulation may comprise not only the active ingredient mentioned above, but also various known excipients and/or additives as appropriate for each dosage form as long as the effect of the above active ingredient is not impaired. In the case of various types of injections, examples of excipients and/or additives include water, glycerol, propylene glycol, and aliphatic polyalcohols such as polyethylene glycol, etc.

The dose (daily dose) of such a parenteral formulation is not limited in any way. For example in the case of various types of injections, the above active ingredient (antibody) is generally used at a dose of preferably 1 to 15 mg/day, more preferably 2 to 12 mg/day, per kg body weight of a subject (patient) to be applied.

For use as an oral formulation, the pharmaceutical composition of the present invention may usually be formulated into any dosage form, such as tablets, capsules, granules, powders, pills, troches, solutions for internal use, suspensions, emulsions, syrups, etc., or may be formulated into a dried product which is dissolved again before use. For use as a pharmaceutical composition, the cancer therapeutic agent of the present invention may optionally comprise pharmaceutically acceptable additives. Specific examples of pharmaceutically acceptable additives include, but are not limited to, antioxidants, preservatives, colorants, flavors, diluents, emulsifiers, suspending agents, solvents, fillers, extenders, buffering agents, delivery vehicles, diluents, carriers, excipients and/or pharmaceutical adjuvants, etc.

In the case of administering siRNA or shRNA, its effective dose is not limited in any way as long as it is sufficient to cause RNAi-mediated degradation of target mRNA. Those skilled in the art would be able to easily determine the effective dose to be administered to a subject in consideration of factors such as the body height, body weight, age and sex of the subject, the route of administration, or the mode of administration, either topical or systemic, etc. In general, siRNA or shRNA is used at a dose of about 0.1 to 1.5 mg/kg for parenteral administration (e.g., intravenous injection).

### EXAMPLES

The present invention will be further described in more detail by way of the following examples, although the present invention is not limited only to these examples.

### [Example 1]

### Analysis of SLC6A6 gene expression

In this example, a novel cancer cell-specific membrane protein was identified, and a novel membrane protein marker was identified with the aim of preparing an antibody for diagnostic or therapeutic purposes. DNA microarrays were used to identify genes which were expressed in common in five lines of cultured colorectal cancer cells (HT29, HCT116, DLD1, LOVO, SW480) and were not expressed in two lines of normal cells (i.e., exfoliated normal colorectal cells derived from two normal subjects receiving colonoscopy).

The resulting 180 candidate genes were narrowed down to 25 genes showing a 2-fold or more difference between cancer lesions of five cases and normal sites by quantitative PCR assay. Further, from among these genes, several genes which were transcribed specifically in cancer lesions were identified by *in situ* hybridization assay. Among them, genes whose expression was observed in none of 39 types of normal tissues publicly available on the database of the Laboratory for Systems Biology and Medicine, the University of Tokyo (http://www.lsbm.org/) were searched to identify a gene for solute carrier family 6 (neurotransmitter transporter, taurine, SLC6A6).

To confirm the expression of the SLC6A6 gene in cancer lesions and normal sites, a probe was prepared against the sequence located at positions 5461-5878 (418 bp) of mRNA (NM_003043) and used for tissue analysis by *in situ* hybridization assay.

Paraffin sections of colorectal cancer tissue (Genostaff Co., Ltd.) were treated with xylene and then rehydrated sequentially with ethanol and PBS, and fixed with paraformaldehyde for 15 minutes. The sections were treated with 7 µg/ml Protreinase K (Roche) for 30 minutes and fixed again with a 4% paraformaldehyde solution. After acetylation with 0.25% acetic anhydride in 0.1 M Tris-HCl pH 8.0 for 10 minutes, the sections were dehydrated with ethanol. The sections were reacted with a hybridization reaction solution (Genostqaff Co., Ltd.) containing 300 ng/ml probe (Genostqaff Co., Ltd.) at 60°C for 16 hours and then washed with 5 × washing solution (Genostqaff Co., Ltd.) at 60°C for 20 minutes and with 50% formamide in 2 × washing solution at 60°C for 20 minutes, followed by treatment with RNaseA at 37°C for 30 minutes.

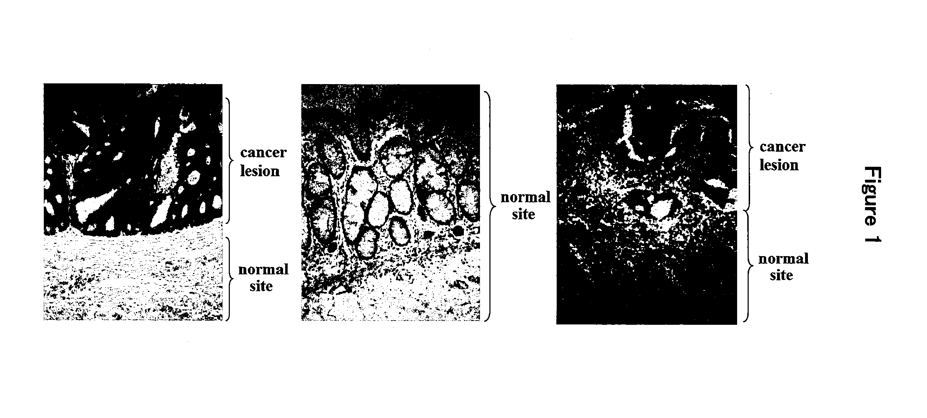

After washing with 2 × washing solution and TBST, the sections were reacted sequentially with 0.5% blocking reaction solution (Roche) and 20% heat-treated sheep serum (Sigma). The sections were reacted with AP-labeled anti-DIG antibody (Roche) for 2 hours and washed with PBS, followed by color development in NBT/BCIP solution (Roche). After counter staining with Kernechtrot solution (Mutoh), the sections were dehydrated and embedded in Marinol (Mutoh) and then observed under a microscope. The results obtained are shown in Figure 1. As shown in Figure 1, cancer lesions of colorectal cancer were specifically stained in comparison with normal sites.

### [Example 2]

### Preparation of monoclonal antibody

### (1) Cells

MCF7-14 was subcultured from Accession No. FERM BP-10944. Caco-2, COLO201, DLD-1, HCT15, HCT116, HT-29, LOVO, SW480, SW620, WiDr and 293T were obtained from the National Cancer Center Research Institute East. Cos7 cells were obtained from Tokyo University of Science.

Culture and subculture were conducted at 37°C under 5% CO₂ for 48 to 72 hours in RPMI 1640 medium (Sigma) containing 10% (v/v) serum (Hyclone) for MCF7-14, SW620, DLD-1 and Colo201, in E-MEM medium (Sigma) for WiDr, in McCoy's 5A medium (Sigma) for HCT116, and in DMEM medium (Sigma) for HT-29, LoVo, SW480, 293T and the other cells, such that cell confluency did not exceed 80% in each case.

### (2) Cloning of SLC6A6 gene

MCF7-14 was cultured and total RNA was extracted with a Qiagen RNeasy Mini kit. From the extracted total RNA (2 µg), cDNA was synthesized by reverse transcription (RT) reaction at 50°C for 1 hour with SuperScript III reverse transcriptase (Invitrogen), followed by heating at 85°C for 5 minutes to stop the reaction. The resulting cDNA was used as a template for PCR reaction with the following primers.
Primer sequences
Forward: AAAGGATCCATGGCCACCAAGGAGAAGCTGC (SEQ ID NO: 5)
Reverse: AAATCTAGACATCATGGTCTCCACAATGATGTG (SEQ ID NO: 6)

The PCR reaction was accomplished by preincubation at 95°C for 10 minutes and subsequent 40 cycles of denaturation at 95°C for 15 seconds and annealing/elongation at 60°C for 1 minute to amplify a gene fragment. The resulting amplified fragment was integrated into pEF6 vector (Invitrogen) by means of the restriction enzymes (BamHI and XbaI) located on the primers. The amplified fragment was confirmed by DNA sequencing, indicating that the same gene sequence as found in the database was integrated and a c-myc tag sequence was added to the C-terminal end.

### (3) Preparation of SLC6A6-overexpressing strains

The plasmid prepared in (2) above was transformed into MCF7-14 cells using FUGENE 6 (Roche). Operations were conducted as described in the manufacturer's instructions attached to this kit.

The cells were cultured in the medium of Example 2(1) supplemented with blasticidin S hydrochloride at 10 µg/mL, and the medium was replaced every 3 to 5 days to select drug-resistant cells. For selection of SLC6A6-overexpressing cells from among the resulting resistant strains, the cultured MCF7-14 cells and the transformed cells were each seeded in 96-well plates at 80% confluency and cultured at 37°C under 5% CO₂ for 16 hours.

After removing the culture supernatant from each well, a 10% (v/v) neutral buffered formalin solution (WAKO) was added in 100 µL volumes and reacted for 10 minutes at room temperature. After removing the formalin solution, the plates were washed three times with PBS(-) and then air-dried to thereby prepare cell-immobilized plates for the respective cases.

Anti-c-myc antibody (santa cruz, clone 9E10) was diluted to 1 µg/mL with TBS-T (25 mM Tris, 150 mM NaCl, 0.05% (v/v) Tween 20, pH 7.4) and added as a primary antibody to the immobilized plates in a volume of 100 µL per well, followed by reaction at room temperature for 1 hour. Each well was washed three times with 200 µL TBS-T.

For use as a secondary antibody, anti-mouse IgG polyclonal antibody-HRP label (BETHYL) was diluted 5,000-fold with TBS-T. The above antibody dilution was added in a volume of 100 µL per well and reacted at room temperature for 30 minutes. Each well was washed three times with 200 µL TBS-T.

Orthophenylenediamine (Sigma) was diluted with 50 mM carbonate-citrate buffer (pH 5.0) to give a final concentration of 0.5 mg/mL and mixed with 1/10,000 volumes of 35% (w/w) aqueous hydrogen peroxide (WAKO). The resulting mixture was added as a substrate solution in a volume of 100 µL per well and reacted at room temperature for 10 minutes. 25 µL of 3 N sulfuric acid (WAKO) was added to stop the reaction. The absorbance at 492 nm was measured with a plate reader (SpectraMax Plus 384, Molecular Devices) to observe signals, thereby selecting three strains showing higher signals than MCF7-14.

### (4) Western blot

Cells of the three strains obtained in (3) above were cultured in 10 cm dishes to reach 90% confluency and washed twice with PBS(-) (0.01 M sodium-phosphate buffer, 0.138 M NaCl, 0.0027 M KCl, pH 7.4). The cells of each strain were supplemented with 200 µL of 2 × RIPA Buffer (0.1 M Tris, 0.3 M EDTA, 1% (v/v) Triton X-100, 2% (w/v) sodium deoxycholate, 0.2% (w/v) sodium dodecyl sulfate) and allowed to stand on ice for 1 minute, followed by collecting a cell suspension with a scraper. The cells were homogenized with an ultrasonic homogenizer (Branson) for 30 seconds to obtain an extract. Extracts were prepared for the respective strains and measured for their protein concentration by the Bradford assay, and then adjusted to the same protein amount and provided for SDS-PAGE. After electrophoresis, proteins were transferred onto a PVDF membrane (PIERCE) with a Trans-Blot SD cell (BioRad Laboratories) in accordance with the manufacturer's recommended protocols. Skimmed milk dissolved at a concentration of 5% (w/v) in TBS-T was used to block the membrane at room temperature for 30 minutes, and the membrane was washed twice with TBS-T. Anti-c-myc antibody was diluted to 1 µg/mL with TBS-T and reacted with the membrane for 1 hour at room temperature. After washing three times with TBS-T, anti-mouse IgG polyclonal antibody-HRP label (BETHYL) was diluted 10,000-fold with TBS-T for use as a secondary antibody. This dilution was reacted with the membrane at room temperature for 30 minutes, followed by washing three times with TBS-T. The membrane was soaked in Immobilon (Millipore) and then wrapped, followed by signal detection with LAS-3000 (Fuji Photo Film Co., Ltd., Japan).

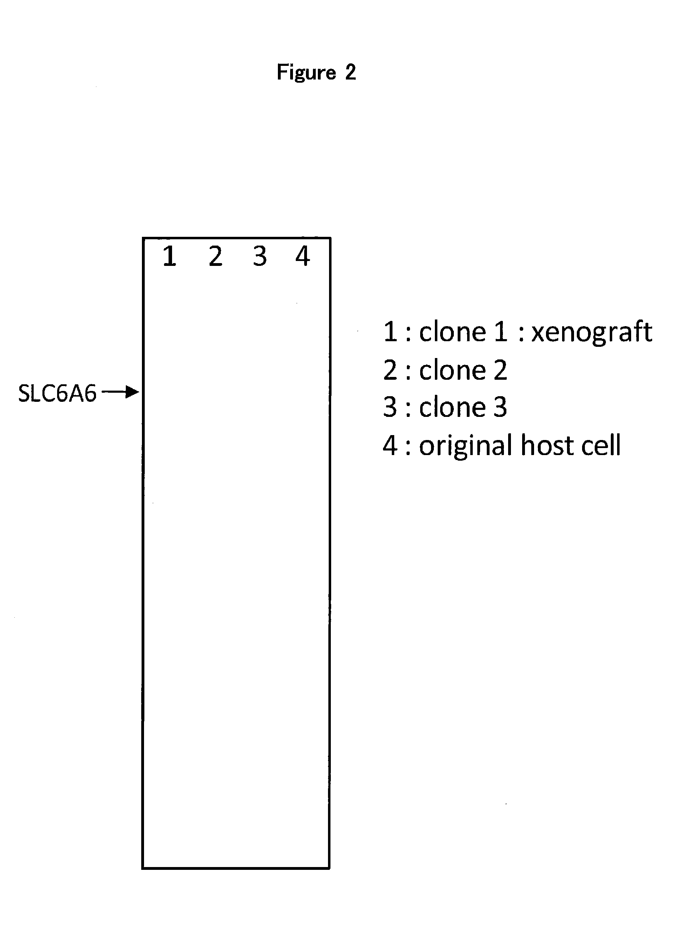

The results obtained are shown in Figure 2. Among the cells transformed with the SLC6A6 gene, a clone found to show highest expression was used for immunization purposes.

### (5) Transplantation of cells

Cells cultured in a 10 cm dish to reach 90% confluency were collected with trypsin (GIBCO) and washed twice with PBS(-) (0.01 M sodium-phosphate buffer, 0.138 M NaCl, 0.0027 M KCl, pH 7.4). The washed cells were suspended in growth factor reduced Matrigel (Becton Dickinson) to give a final density of 8.6 × 10⁷ cells/mL and stored on ice before use in transplantation.

Chloral hydrate (Sigma) was dissolved at a concentration of 3.5% (w/v) in physiological saline to prepare a 3.5% solution of chloral hydrate in physiological saline. Nude mice at 6 to 8 weeks of age (BALB/cALcl-nu/nu line (CLEA Japan, Inc., Japan)) were anesthetized by being intraperitoneally administered with 0.2 mL of the 3.5% solution of chloral hydrate in physiological saline. Into the fourth mammary glands in each mouse, the cells suspended in the Matrigel were transplanted at 1 × 10⁶ cells per mammary gland via a 24G injection needle, such that the cells did not extend off the mammary gland. Each mouse received two transplantations, one at left and another at right fourth mammary gland in the trunk.

### (6) Expression and purification of SLC6A6 partial protein for screening

From the full-length gene for SLC6A6 introduced into pEF6 vector in Example 2(3), DNA (SEQ ID NO: 3) encoding a region covering amino acid residues 143 to 216 (SEQ ID NO: 4) in the extracellular region was subcloned into pET32 vector. The following primers were used for PCR.
Primer sequences
Forward: ATAGGATCCGGCCTGGGCCACATATCACCTG (SEQ ID NO: 7)
Reverse: TATGAATTCGCTTTCAGAGAGCCTGGGTGGTC (SEQ ID NO: 8)

The PCR reaction was accomplished by preincubation at 94°C for 2 minutes and subsequent 30 cycles of denaturation at 98°C for 10 seconds, annealing at 58°C for 30 seconds and elongation at 68°C for 30 seconds to amplify a gene fragment.

The resulting amplified fragment was integrated into pET32 vector (Novagen) by means of the restriction enzymes (EcoRI and BamHI) located on the primers.

The amplified fragment was confirmed for its nucleotide sequence by DNA sequencing, indicating that the same extracellular region gene sequence as found in the database was integrated and a His tag sequence was added to the C-terminal end. BL21(DE3) (Invitrogen) was transformed with this vector and cultured in LB medium (1% (w/v) tryptone (Sigma), 0.5% (w/v) yeast extract (Sigma), 0.5% (w/v) NaCl (Sigma)) supplemented with 1% (w/v) glucose. After the medium turbidity reached 0.6 at a wavelength of 600 nm, 1 mM IPTG (WAKO) as added and culture was continued for 16 hours. The microbial cells were collected by centrifugation and then homogenized by ultrasonication to obtain a fraction containing the extracellular region of SLC6A6 as an insoluble protein.

About 10 mg of the sample was dissolved in Buffer A (1 M guanidine hydrochloride (Sigma), 10 mM DTT (Sigma), 10 mM EDTA (Sigma)) and reacted at 37°C for 1 hour. 1 L of Buffer B (50 mM Tris, 150 mM NaCl, 5% glycerol, 0.4 mM oxidized glutathione (Sigma), pH 8.5) was added gently, followed by stirring at 4°C for 18 hours. The dissolved sample was applied to a Ni sepharose column (GE) and eluted with Buffer C (50 mM potassium phosphate buffer, 150 mM NaCl, 200 mM imidazole, pH 8.0), followed by dialysis against imidazole-free Buffer C to obtain a partial protein of the extracellular region of SLC6A6 in a purified state.

### (7) Antiserum analysis

The recombinant protein obtained in (6) above (10 µg/ml) was dispensed in 100 µL volumes into MaxiSorp 96-well plates (Nunc) and adsorbed onto the plates at room temperature for 1 hour. After adsorption, each well was washed with TBS-T (25 mM Tris, 150 mM NaCl, 0.05% (v/v) Tween 20, pH 7.4) and charged with skimmed milk (GIBCO), which had been diluted at a concentration of 5% with TBS-T, to conduct blocking for 30 minutes at room temperature. After each well was washed three times with 200 µL TBS-T, mouse plasma samples collected from the tail vein were each diluted 1/2000-fold with TBS-T and added to the ELISA plates in a volume of 100 µL per well, followed by reaction at room temperature for 1 hour. Each well was washed three times with 200 µL TBS-T.

For use as a secondary antibody, anti-mouse IgG polyclonal antibody-HRP label (BETHYL) was diluted 5,000-fold with TBS-T. The above antibody dilution was added in a volume of 100 µL per well and reacted at room temperature for 30 minutes. Each well was washed three times with 200 µL TBS-T. Orthophenylenediamine (Sigma) was diluted with 50 mM carbonate-citrate buffer (pH 5.0) to give a final concentration of 0.5 mg/mL and mixed with 1/10,000 volumes of 35% (w/w) aqueous hydrogen peroxide (WAKO). The resulting mixture was added as a substrate solution in a volume of 100 µL per well and reacted at room temperature for 10 minutes. 25 µL of 3 N sulfuric acid (WAKO) was added to stop the reaction. The absorbance at 492 nm was measured with a plate reader (SpectraMax Plus 384, Molecular Devices) to analyze antibody titers, which were then used for selection of mice to be used for cell fusion.

### (8) Cell fusion

Mouse spleen lymphocytes were electrically fused with mouse myeloma cell line P3X63-Ag8 (ATCC Accession No. CRL-1580). For cell fusion, 1 × 10⁸ spleen cells were mixed with 0.25 × 10⁸ cells of the myeloma cell line and suspended in EP Buffer (0.3 M mannitol, 0.1 mM CaCl₂, 0.1 mM MgCl₂) to give a cell density of 0.25 × 10⁸ cells/mL, followed by cell fusion with an electro cell fusion generator LF201 (Nepa Gene Co., Ltd., Japan). Fusion conditions were set in accordance with the manufacturer's recommended protocols.

The fused cells were suspended in HAT medium (Invitrogen) and dispensed into thirty 96-well plates in a volume of 100 µL per well. During culture, 200 µL of HAT medium was added to each well. After culture for 11 to 16 days, the plates were observed under a microscope, indicating that 5 to 12 colonies were formed per well.

### (9) Obtaining monoclonal antibodies

At 7 months after transplantation, spleen cells were collected and used to prepare hybridoma cells in the same manner as shown in (8) above. For selection of antibodies recognizing SLC6A6, the same procedures as shown in (3) and (7) above were used to select clones. The culture supernatant of each clone was used as a primary antibody, while anti-mouse IgG polyclonal antibody-HRP label was used as a secondary antibody.

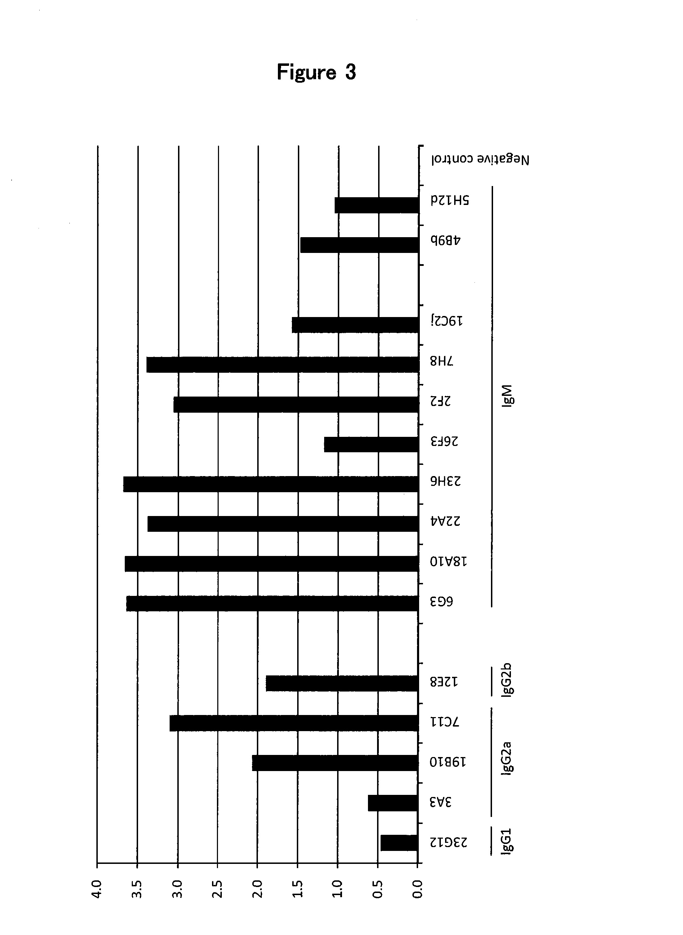

Figure 3 shows the results analyzed for the resulting clones in the same manner as shown in (7) above. "3A3," "19B10," "23G12," "7C11," "12E8," "6G3," "18A10," "22A4," "23H6," "26F3," "2F2," "7H8" and "19C2j" represent clone numbers. Hereinafter, antibodies produced by hybridoma cells are also represented by their respective clone numbers.

### (10) Tissue staining

4B9b antibody and 5H12d antibody were used for immunostaining. Paraffin-embedded slices of human colorectal cancer tissue (Biochain) were deparaffinized with xylene and rehydrated with ethanol. After being treated with 0.3% (v/v) aqueous hydrogen peroxide for 20 minutes, the slices were washed three times with TBST and treated with steam under pressure (120 °C) for 10 minutes to activate antigens. The slices were treated with 3% (w/v) BSA-containing PBS and then reacted with either 4B9b antibody or 5H12d antibody at 4°C for 16 hours. After washing three times with TBST, the slices were reacted with peroxidase-labeled anti-mouse secondary antibody (DAKO) at room temperature for 1 hour and washed three times with TBST, followed by color development with DAB reagent (DAKO) for 5 minutes. After washing with distilled water, nuclei were stained with Hematoxylin (WAKO), and the slices were washed with running water, treated sequentially with ethanol and xylene, and then embedded.

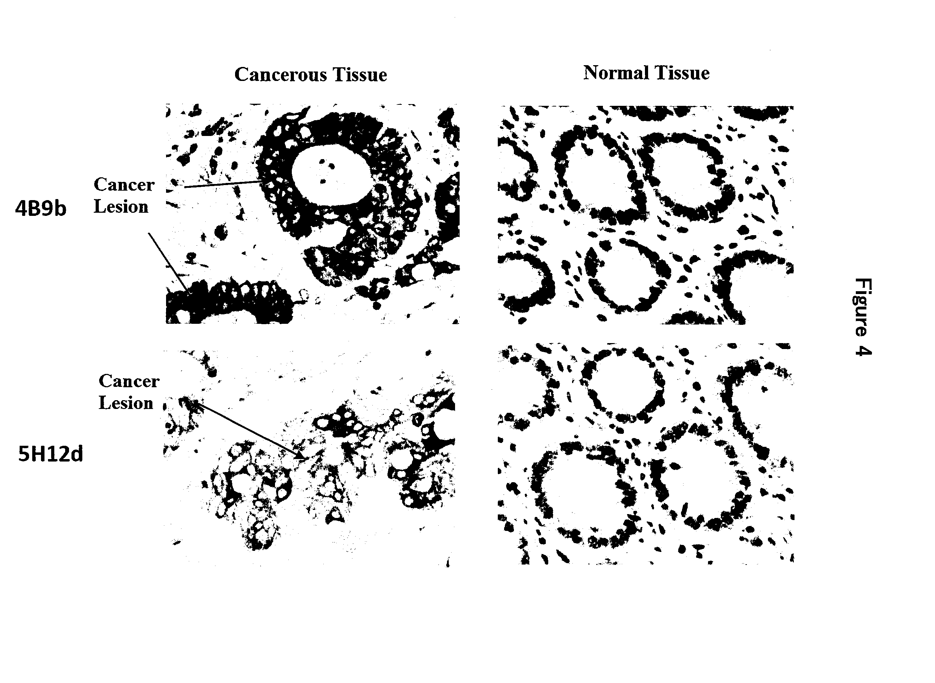

Figure 4 shows the results of immunostaining performed on cancer lesions and normal sites in a colorectal cancer patient. The results indicated that cancer lesions were stained specifically.

### (11) Tissue staining of normal tissues

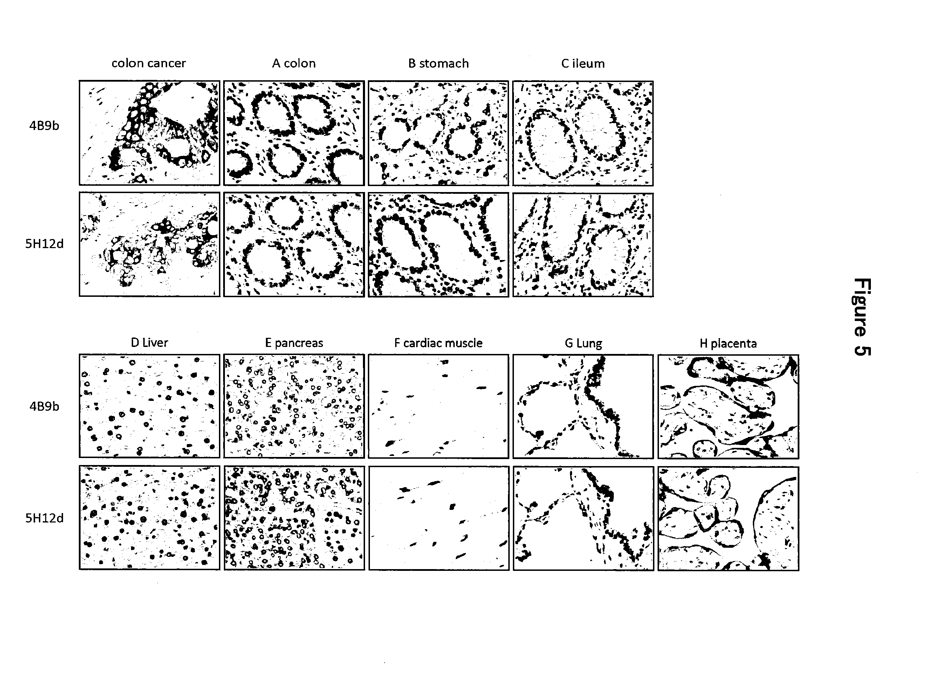

4B9b and 5H12d were used for immunostaining of human normal tissues (Figure 5). The same procedures as shown in (10) were repeated for this purpose.

In Figure 5, panels A to H represent A: colon, B: stomach, C: ileum, D: liver, E: pancreas, F: cardiac muscle, G: lung and H: placenta, respectively. Since the antibodies used were IgM, backgrounds were observed in some degree, although the cell membrane was stained only in the case of colorectal cancer.

### (12) Real-time quantitative RT-PCR

From the cultured cells, total RNA was extracted with an RNeasy Mini Kit (Qiagen). cDNA was synthesized from the total RNA with a 1st strand cDNA Synthesis Kit (Roche). Real-time quantitative PCR of SLC6A6 cDNA with a LightCycler system (Roche) was conducted in a reaction solution (20 µl) containing LightCyclere DNA Master SYBR Green I (FastStart TaqDNA polymerase, deoxynucleotide triphosphate buffer, SYBR Green I), 3.0 mM MgCl₂ and 0.5 µM of each primer sequence in LightCycler capillaries. The primers used were SLC6A6 TagMan probe Hs00161778 (Applied Biosystems).

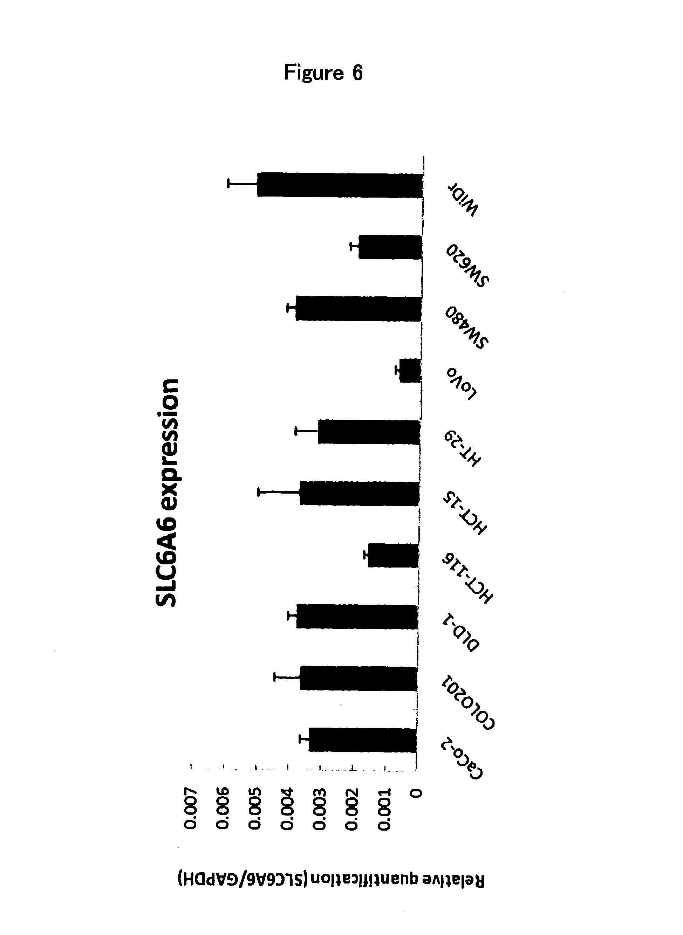

PCR products were optimized in the linear range by serial dilution of template cDNA. Quantitative PCR data were analyzed using LightCycler software version 3.3. The results obtained are shown in Figure 6.

### (13) FACS analysis

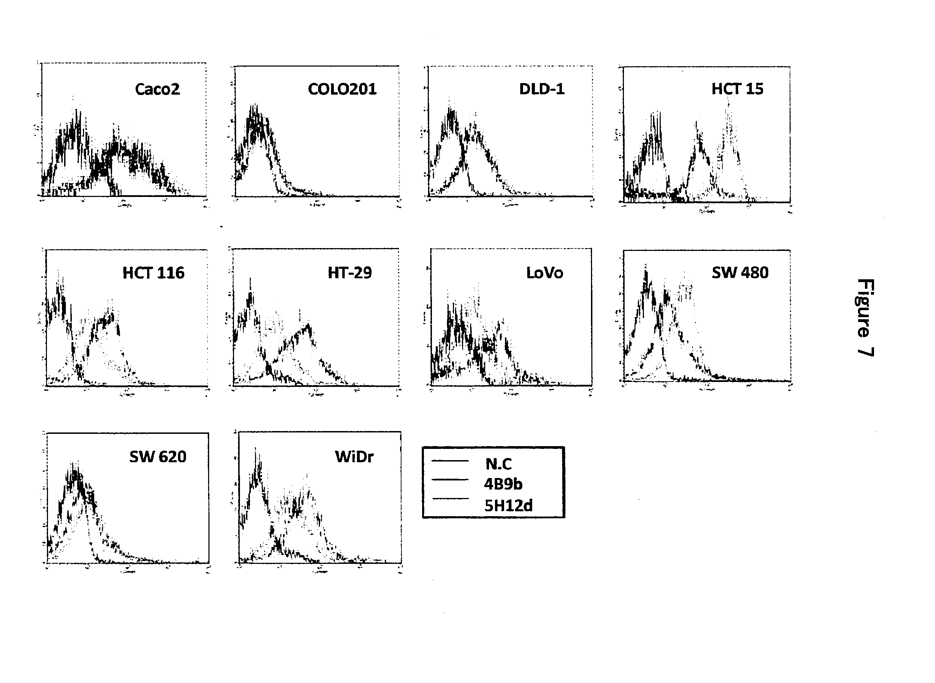

Ten lines of colorectal cancer cells were each cultured to reach 90% confluency. The cells of each line were washed twice with PBS, detached with a scraper and collected into a 1.5 mL tube. 4B9b and 5H12d antibodies were each added to give a final concentration of 1 µg/mL, followed by reaction for 60 minutes. After washing twice with PBS + 2% FBS, AlexaFluor 488-labeled goat anti-mouse IgG (Invitrogen) was added as a 1/1000 dilution in PBS + 2% FBS, followed by reaction for 30 minutes. After washing twice with PBS + 2% PBS(FBS?), the cells were analyzed by Guava Flow Cytometry (Millipore). The results obtained are shown in Figure 7.

As can be seen from Figure 7, the antibodies of the present invention were found to react with all lines of colorectal cancer cells.

### [Example 3]

### (1) Construction of mouse IgG conversion gene

From hybridoma cells expressing 4B9b antibody, total RNA was extracted with an RNeasy Mini Kit and cDNA was synthesized with a 1st strand cDNA Synthesis Kit. KODPlus (TOYOBO) was used as an enzyme for PCR and the experiment was conducted in accordance with the manufacturer's recommended protocols unless otherwise specified. To amplify an H chain variable region fragment of the antibody, the primers shown in SEQ ID NO: 9 and SEQ ID NO: 10 were used in PCR reaction (30 cycles of denaturation at 94°C for 2 minutes, subsequent annealing at 30°C for 15 seconds and elongation at 68°C for 45 seconds) to thereby obtain the desired fragment.
VH_BACK: AAGTSMARCTGCAGSAGTCWGG (SEQ ID NO: 9)
Bi4m: GGAGACGAGGGGGAAAAGCTTTGGGAAGGACTGACTCTC (SEQ ID NO: 10)

To amplify an L chain variable region fragment of the antibody, the primers shown in SEQ ID NO: 11 and SEQ ID NO: 12 were used in PCR reaction (30 cycles of denaturation at 94°C for 2 minutes, subsequent annealing at 30°C for 15 seconds and elongation at 68°C for 30 seconds) to thereby obtain the desired fragment.
LBACK: GACATTCAGCTGACCCAGTCTCCA (SEQ ID NO: 11)
VLFOR: GTTAGATCTCCAGCTTGGTCCC (SEQ ID NO: 12)

The purified PCR amplification fragments were each mixed with 10 mM dNTP Mix (Invitrogen) and 2 × GoTaq Maxter Mix (Promega) and reacted at 70°C for 15 minutes, followed by ice cooling at 4°C for 2 minutes to ensure dA addition to the 3'-terminal end. Then, using a pGEM-T-Easy Vector System (Promega), the H chain fragment and the L chain fragment were cloned by the so-called TA cloning method.

### (i) Construction of H chain

Subsequently, for addition of a signal sequence, total RNA was extracted in the same manner as shown above from CHO cells expressing human-mouse chimeric IgG1 antibody (obtained from the department of countermeasures to cancer therapy, the National Cancer Center) and reverse-transcribed into cDNA, followed by PCR reaction (30 cycles of denaturation at 94°C for 2 minutes, subsequent annealing at 58°C for 15 seconds and elongation at 68°C for 15 seconds) with the primers shown in SEQ ID NO: 13 and SEQ ID NO: 14 to thereby obtain the desired fragment.
chimera_H_signal_F: ATGGCTTGGGTGTGGACCTTGC (SEQ ID NO: 13)

chimera_H_signal+4B9b_VH_R: CCAGACTGCTGCAGTTTGACCTGTGCTTGGG
CACTTTGGGC (SEQ ID NO: 14)

Concurrently, total RNA was also extracted from hybridoma cells expressing mouse IgG1 (already established anti-mouse albumin monoclonal antibody C, clone ALB1) and reverse-transcribed into cDNA, followed by PCR reaction (30 cycles of denaturation at 94°C for 2 minutes, subsequent annealing at 58°C for 15 seconds and elongation at 68°C for 15 seconds) with the primers shown in SEQ ID NO: 15 and SEQ ID NO: 16 to thereby obtain the desired fragment. 
4B9b_VH+mCg1_F: CTCTGGTCACTGTCTCTGCAGCCAAAACGACACCCCCAT
CTG (SEQ ID NO: 15)
 mCg1_R: TCATTTACCAGGAGAGTGGGAGAG (SEQ ID NO: 16)

The thus prepared signal sequence, variable region and constant region of the antibody H chain were ligated by PCR reaction. The signal sequence, variable region and constant region were mixed together and provided for PCR reaction (32 cycles of denaturation at 94°C for 2 minutes, subsequent annealing at 58°C for 30 seconds and elongation at 68°C for 1 minute) with the primers shown in SEQ ID NO: 13 and SEQ ID NO: 16 to thereby obtain a ligated fragment.

These fragments thus ligated were cloned into pGEM-T-Easy Vector in the same manner as above for TA cloning, and then cloned into pcDNA3.1-myc/HisA (Invitrogen) by means of the restriction enzyme (NotI) located at the 5'-side of the signal sequence and the 3'-side of the constant region.

### (ii) Construction of L chain

As in the case of the H chain, a signal sequence, a variable region and a constant region were each cloned and ligated together to obtain a fragment for L chain.

For addition of a signal sequence, the primers shown in SEQ ID NO: 17 and SEQ ID NO: 18 were used in PCR reaction (preincubation at 94°C for 2 minutes, 30 cycles of denaturation at 94°C for 15 seconds, subsequent annealing at 58°C for 15 seconds and elongation at 68°C for 30 seconds) to thereby obtain the desired fragment.

chimera_L_signal_F(KpnI): GGCTTCATGGTGCTCAGTGTGACATTCAGCTGAC
CCAGT (SEQ ID NO: 17)

chimera_L_signal+4B9b_VL_R: AGTTGGTGCAGCATCAGCCCGTTTGATCTCCA
GCTTGGTC (SEQ ID NO: 18)

For cloning of a constant region, the primers shown in SEQ ID NO: 19 and SEQ ID NO: 20 were used in PCR reaction (preincubation at 94°C for 2 minutes, 30 cycles of denaturation at 94°C for 15 seconds, subsequent annealing at 58°C for 15 seconds and elongation at 68°C for 30 seconds) to thereby obtain the desired fragment. 
4B9b_VL+mCk_F: GACCAAGCTGGAGATCAAACGGGCTGATGCTGCACCAAC
T (SEQ ID NO: 19)
 mCk_R(EcoRI): TTTGAATTCCTAACACTCATTCCTGTTGAAG (SEQ ID NO: 20)

As in the case of the H chain, the signal sequence, variable region and constant region were mixed together and ligated by PCR reaction with the primers shown in SEQ ID NO: 17 and SEQ ID NO: 20. These fragments thus ligated were cloned into pEF6 vector (Invitrogen) by means of the restriction enzymes (KpnI and EcoRI) designed on the primers. The H chain of the mouse IgG1-converted anti-SLC6A6 antibody has the DNA sequence and amino acid sequence shown in SEQ ID NO: 21 and SEQ ID NO: 22, respectively. Likewise, the L chain has the DNA sequence and amino acid sequence shown in SEQ ID NO: 23 and SEQ ID NO: 24, respectively.

SEQ ID NO: 21:
ATGGCTTGGGTGTGGACCTTGCTACTCCTGATGGCAGCTGCCCAAAGTGCCC
AAGCACAGGTCAAACTGCAGCAGTCTGGAGCTGAGCTGATGAAGCCTGGGG
CCTCAGTGAAGATATCCTGCAAGGCTACTGGCTACACATTCAGTAGGTACTG
GATAGAGTGGGTAAAGCAGAGGCCTGGACATGGCCTTGAGTGGATTGGAGA
GATTTTACCTGGAAGTGGTAGTACTAACTACAATGAGAAGTTCAAGGGCAA
GGCCACATTCACTGCAGATACATCCTCCAACACAGCCTACATGCAACTCAG
CAGCCTGACATCTGAGGACTCTGCCGTCTATTACTGTGCAAGAGGAGGATTT

CTTGGGCATTACGGGTTTGCTTACTGGGGCCAAGGGACTCTGGTCACTGTCT
CTGCAGCCAAAACGACACCCCCATCTGTCTATCCACTGGCCCCTGGATCTGC
TGCCCAAACTAACTCCATGGTGACCCTGGGATGCCTGGTCAAGGGCTATTTC
CCTGAGCCAGTGACAGTGACCTGGAACTCTGGATCCCTGTCCAGCGGTGTG
CACACCTTCCCAGCTGTCCTGCAGTCTGACCTCTACACTCTGAGCAGCTCAG
TGACTGTCCCCTCCAGCACCTGGCCCAGCGAGACCGTCACCTGCAACGTTGC
CCACCCGGCCAGCAGCACCAAGGTGGACAAGAAAATTGTGCCCAGGGATTG
TGGTTGTAAGCCTTGCATATGTACAGTCCCAGAAGTATCATCTGTCTTCATC
TTCCCCCCAAAGCCCAAGGATGTGCTCACCATTACTCTGACTCCTAAGGTCA
CGTGTGTTGTGGTAGACATCAGCAAGGATGATCCCGAGGTCCAGTTCAGCT
GGTTTGTAGATGATGTGGAGGTGCACACAGCTCAGACGCAACCCCGGGAGG
AGCAGTTCAACAGCACTTTCCGCTCAGTCAGTGAACTTCCCATCATGCACCA
GGACTGGCTCAATGGCAAGGAGTTCAAATGCAGGGTCAACAGTGCAGCTTT
CCCTGCCCCCATCGAGAAAACCATCTCCAAAACCAAAGGCAGACCGAAGGC
TCCACAGGTGTACACCATTCCACCTCCCAAGGAGCAGATGGCCAAGGATAA
AGTCAGTCTGACCTGCATGATAACAGACTTCTTCCCTGAAGACATTACTGTG
GAGTGGCAGTGGAATGGGCAGCCAGCGGAGAACTACAAGAACACTCAGCC
CATCATGGACACAGATGGCTCTTACTTCGTCTACAGCAAGCTCAATGTGCAG
AAGAGCAACTGGGAGGCAGGAAATACTTTCACCTGCTCTGTGTTACATGAG
GGCCTGCACAACCACCATACTGAGAAGAGCCTCTCCCACTCTCCTGGTAAAT
GA

SEQ ID NO: 22:
MAWVWTLLLLMAAAQSAQAQVKLQQSGAELMKPGASVKISCKATGYTFSRY
WIEWVKQRPGHGLEWIGEILPGSGSTNYNEKFKGKATFTADTSSNTAYMQLSS
LTSEDSAVYYCARGGFLGHYGFAYWGQGTLVTVSAAKTTPPSVYPLAPGSAA
QTNSMVTLGCLVKGYFPEPVTVTWNSGSLSSGVHTFPAVLQSDLYTLSSSVTVP
SSTWPSETVTCNVAHPASSTKVDKKIVPRDCGCKPCICTVPEVSSVFIFPPKPKD
VLTITLTPKVTCVVVDISKDDPEVQFSWFVDDVEVHTAQTQPREEQFNSTFRSV
SELPIMHQDWLNGKEFKCRVNSAAFPAPIEKTISKTKGRPKAPQVYTIPPPKEQM
AKDKVSLTCMITDFFPEDITVEWQWNGQPAENYKNTQPIMDTDGSYFVYSKLN
VQKSNWEAGNTFTCSVLHEGLHNHHTEKSLSHSPGK*

SEQ ID NO: 23:
ATGAGACCGTCTATTCAGTTCCTGGGGCTCTTGTTGTTCTGGCTTCATGGTGC
TCAGTGTGACATTCAGCTGACCCAGTCTCCAACCACCATGGCTGCATCTCCC
GGGGAGAAGATCACTATCACCTGCAGTGCCAGCTCAAGTATAAGTTCCAAT
TACTTGCATTGGTATCAGCAGAAGCCAGGATTCTCCCCTAAACTCTTGATTT
ATAGGACATCCAATCTGGCTTCTGGAGTCCCAGCTCGCTTCAGTGGCAGTGG
GTCTGGGACCTCTTACTCTCTCACAATTGGCACCATGGAGGCTGAAGATGTT
GCCACTTACTACTGCCAGCAGGGTAGTAGTATACCGTACACGTTCGGAGGG

GGGACCAAGCTGGAGATCAAACGGGCTGATGCTGCACCAACTGTATCCATC
TTCCCACCATCCAGTGAGCAGTTAACATCTGGAGGTGCCTCAGTCGTGTGCT
TCTTGAACAACTTCTACCCCAAAGACATCAATGTCAAGTGGAAGATTGATG
GCAGTGAACGACAAAATGGCGTCCTGAACAGTTGGACTGATCAGGACAGCA
AAGACAGCACCTACAGCATGAGCAGCACCCTCACGTTGACCAAGGACGAGT
ATGAACGACATAACAGCTATACCTGTGAGGCCACTCACAAGACATCAACTT
CACCCATTGTCAAGAGCTTCAACAGGAATGAGTGTTAG

SEQ ID NO: 24:
MRPSIQFLGLLLFWLHGAQCDIQLTQSPTTMAASPGEKITITCSASSSISSNYLHW
YQQKPGFSPKLLIYRTSNLASGVPARFSGSGSGTSYSLTIGTMEAEDVATYYCQ
QGSSIPYTFGGGTKLEIKRADAAPTVSIFPPSSEQLTSGGASVVCFLNNFYPKDIN
VKWKIDGSERQNGVLNSWTDQDSKDSTYSMSSTLTLTKDEYERHNSYTCEAT
HKTSTSPIVKSFNRNEC

### (2) Expression and purification of mouse IgG-converted antibody

Cos7 cells were cultured in four 10 cm dishes to reach 90% confluency, and the H chain and L chain expression vectors prepared in (1) were transformed into the Cos7 cells using Fugene 6 (Roche) to cause transient protein expression. Transformation conditions were set in accordance with the manufacturer's recommended protocols. After 24 hours, the cells were transferred for subculture to twenty 10 cm dishes and cultured for 6 days in a medium supplemented with 10 µg/mL blasticidin (Invitrogen) and 800 µg/mL G418 (Nacalai Tesque, Inc., Japan). The culture supernatants were collected and purified with a Protein G column. The Protein G column (GE Healthcare) was used in a volume of 1 mL relative to 200 mL of the culture supernatant of the transformed Cos7 cells. The cultured solution was passed at a flow rate of 1 to 3 ml/min through the Protein G column which had been equilibrated with PBS, followed by washing with 6 mL of washing buffer (25 mM Tris-HCl (pH 7.4), 140 mM NaCl, 10 mM KCl). Then, antibody proteins were eluted with 1 mL of elution buffer (0.1 M glycine (pH 2.5)) and neutralized with 3 M Tris-HCl (pH 7.4) to be within pH 7.0 to 7.4. The antibody proteins were concentrated with Amicon Ultra 30 (Millipore) and the buffer was replaced with PBS.

### (3) Activity measurement by ELISA

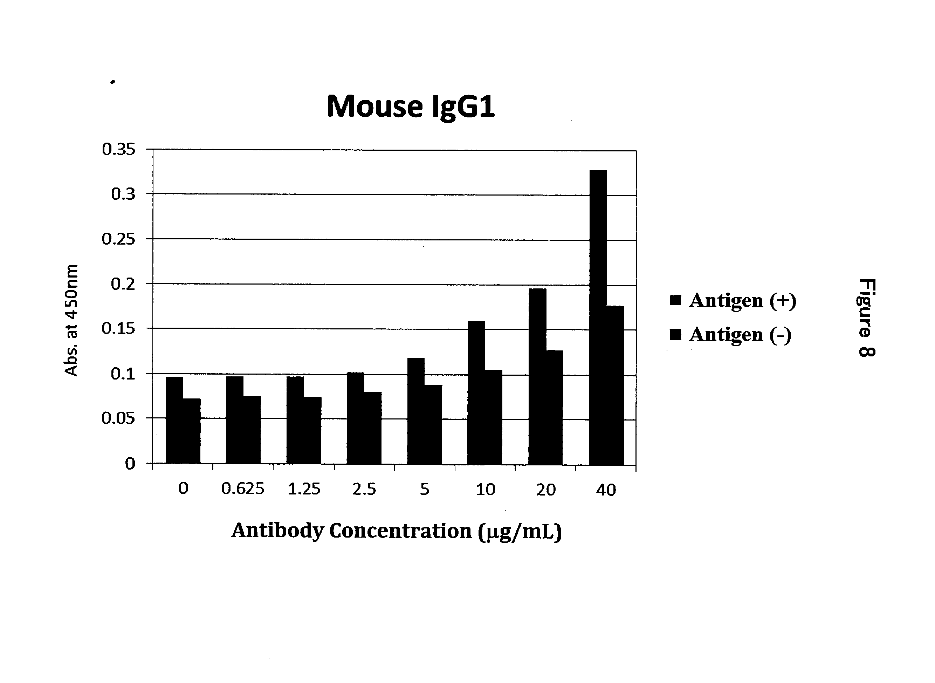

Activity measurement was conducted in the same manner as shown in Example 2(7). In this measurement, the antibody concentration was set to 0 to 40 µg/mL. The results of activity measurement are shown in Figure 8. The mouse IgG-converted antibody was also confirmed to have activity.

### (4) Immunohistological staining

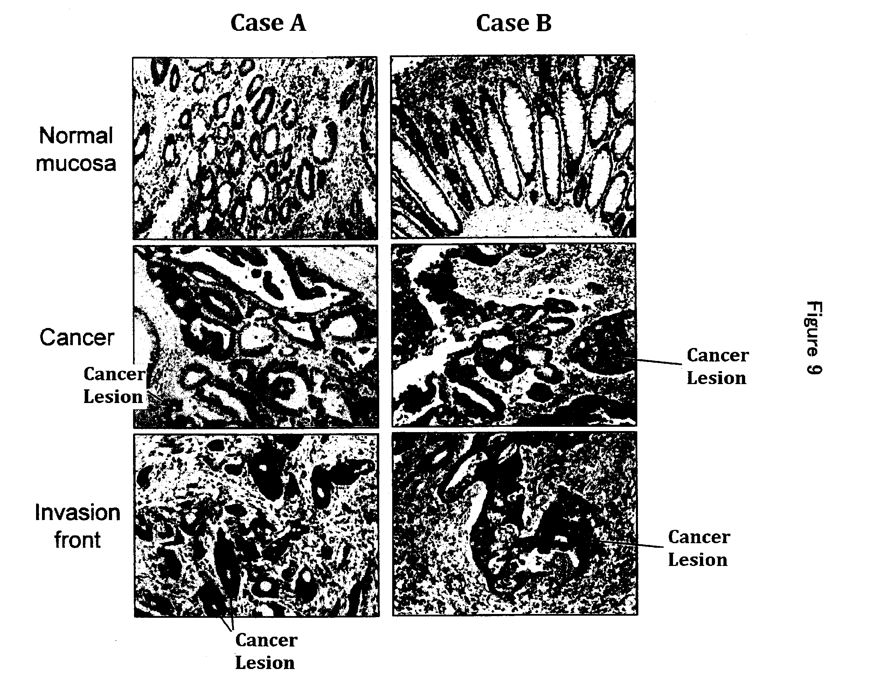

Immunohistological staining was conducted in the same manner as shown in Example 2(11). For tissue samples from three colorectal cancer patients, normal site (normal mucosa), cancer lesion (cancer) and cancer invasion site (invasion front) were stained. The results obtained for two cases (A and B) are shown in Figure 9. The results indicate that the staining becomes clearer in the direction from the normal site to the tumor edge. Namely, the anti-SLC6A6 antibody of the present invention was found to bind to colorectal cancer, in particular strongly bind to invasion sites where colorectal cancer has spread. This suggests that the antibody of the present invention is very advantageous for development of therapeutic antibodies in terms of its ability to suppress cancer through binding to sites where cancer is spreading. Moreover, in addition to the antibody, its target SLC6A6 would also be advantageous as a target for colorectal cancer treatment.

### [Example 4]

### (1) Human IgG conversion

In the same manner as shown in Example 3(1), a signal sequence, a variable region and a constant region were each cloned and then ligated together to prepare an expression vector. For cloning of the human IgG1 constant region and signal sequence, their amplification was conducted from CHO cells expressing human-mouse chimeric IgG1 antibody (obtained from the department of countermeasures to cancer therapy, the National Cancer Center).

### (i) Construction of H chain

The primers shown in SEQ ID NO: 25 and SEQ ID NO: 26 were used for cloning of the H chain variable region and the primers shown in SEQ ID NO: 27 and SEQ ID NO: 28 were used for cloning of the constant region in PCR reaction (preincubation at 94°C for 2 minutes, 32 cycles of denaturation at 94°C for 15 seconds, subsequent annealing at 58°C for 15 seconds and elongation at 68°C for 1 minute) to thereby obtain the desired fragments. 
chimera_H_signal+4B9b_VH_F: GCCCAAAGTGCCCAAGCACAGGTCAAACTGC
AGCAGTCTGG (SEQ ID NO: 25)
 
4B9b_VH+hCg1_R: GATGGGCCCTTGGTGCTAGCTGCAGAGACAGTGACCAG
AG (SEQ ID NO: 26)
 
4B9b_VH+hCg1_F: CTCTGGTCACTGTCTCTGCAGCTAGCACCAAGGGCCCAT
CG (SEQ ID NO: 27)
 hCg1_R(NotI): TTTGCGGCCGCTCATTTACCCGGAGACAGGG (SEQ ID NO: 28) For cloning of the H chain signal sequence, the primers shown in SEQ ID NO: 29 and SEQ ID NO: 30 were used in PCR reaction (preincubation at 94°C for 2 minutes, 32 cycles of denaturation at 94°C for 15 seconds, subsequent annealing at 58°C for 15 seconds and elongation at 68°C for 20 seconds) to thereby obtain the desired fragment.
chimera_H_signal_F(NotI): TTTGCGGCCGCACCATGGCTTGGGTGTGGACCTT (SEQ ID NO: 29)

chimera_H_signal+4B9b_VH_R: CCAGACTGCTGCAGTTTGACCTGTGCTTGGG
CACTTTGGGC (SEQ ID NO: 30)

As in the case of the mouse antibody, the signal sequence, variable region and constant region were ligated by PCR reaction with the primers shown in SEQ ID NO: 25 and SEQ ID NO: 30, and these fragments thus ligated were cloned into pcDNA3.1-myc/HisA vector by means of the restriction enzyme (NotI) designed on the primers.

### (ii) Construction of L chain

As in the case of construction of the mouse IgG1 L chain, a signal sequence, a variable region and a constant region were each cloned and ligated together to obtain a fragment for L chain.

The primers shown in SEQ ID NO: 31 and SEQ ID NO: 32 were used for cloning of the variable region, the primers shown in SEQ ID NO: 33 and SEQ ID NO: 34 were used for cloning of the constant region, and the primers shown in SEQ ID NO:

35 and SEQ ID NO: 36 were used for cloning of the signal sequence in PCR reaction (preincubation at 94°C for 2 minutes, 30 cycles of denaturation at 94°C for 15 seconds, subsequent annealing at 58°C for 15 seconds and elongation at 68°C for 30 seconds) to thereby obtain the desired fragments. 
chimera_L_signal+4B9b_VL_F: GGCTTCATGGTGCTCAGTGTGACATTCAGCTG
ACCCAGTC (SEQ ID NO: 31)
 4B9b_VL+hCk_R: AGATGGTGCAGCCACCGTACGTTTGATCTCCAGCTTGGTC (SEQ ID NO: 32)
4B9b_VL+hCk_F: GACCAAGCTGGAGATCAAACGTACGGTGGCTGCACCATCT (SEQ ID NO: 33)
hCk_R(EcoRI): TTTGAATTCTAACACTCTCCCCTGTTGAAGC (SEQ ID NO: 34) chimera_L_signal_F(KpnI): TTTGGTACCACCATGAGACCGTCTATTCAGTT (SEQ ID NO: 35)

chimera_L_signal+4B9b_VL_R: GACTGGGTCAGCTGAATGTCACACTGAGCAC
CATGAAGCC (SEQ ID NO: 36)

Also in the case of the L chain, the signal sequence, variable region and constant region were ligated by PCR reaction with the primers shown in SEQ ID NO: 31 and SEQ ID NO: 36, and then ligated to the constant region by PCR reaction with the primers shown in SEQ ID NO: 36 and SEQ ID NO: 37. These fragments thus ligated were cloned into pEF6 vector (Invitrogen) by means of the restriction enzymes (KpnI and EcoRI) designed on the primers. The H chain of the mouse(human?) IgG1-converted anti-SLC6A6 antibody is as shown in SEQ ID NO: 37 (nucleotide sequence) and SEQ ID NO: 38 (amino acid sequence), while the L chain is as shown in SEQ ID NO: 39 (nucleotide sequence) and SEQ ID NO: 40 (amino acid sequence).

SEQ ID NO: 37:
TCCTCCAACACAGCCTACATGCAACTCAGCAGCCTGACATCTGAGGACTCTG
CCGTCTATTACTGTGCAAGAGGAGGATTTCTTGGGCATTACGGGTTTGCTTA
CTGGGGCCAAGGGACTCTGGTCACTGTCTCTGCAGCTAGCACCAAGGGCCC
ATCGGTCTTCCCCCTGGCACCCTCCTCCAAGAGCACCTCTGGGGGCACAGCG
GCCCTGGGCTGCCTGGTCAAGGACTACTTCCCCGAACCGGTGACGGTGTCGT
GGAACTCAGGCGCCCTGACCAGCGGCGTGCACACCTTCCCGGCTGTCCTAC
AGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACAC
CAAGGTGGACAAGAAAGTTGAGCCCAAATCTTGTGACAAAACTCACACATG
CCCACCGTGCCCAGCACCTGAACTCCTGGGGGGACCGTCAGTCTTCCTCTTC
CCCCCAAAACCCAAGGACACCCTCATGATCTCCCGGACCCCTGAGGTCACA
TGCGTGGTGGTGGACGTGAGCCACGAAGACCCTGAGGTCAAGTTCAACTGG
TACGTGGACGGCGTGGAGGTGCATAATGCCAAGACAAAGCCGCGGGAGGA
GCAGTACAACAGCACGTACCGTGTGGTCAGCGTCCTCACCGTCCTGCACCA
GGACTGGCTGAATGGCAAGGAGTACAAGTGCAAGGTCTCCAACAAAGCCCT
CCCAGCCCCCATCGAGAAAACCATCTCCAAAGCCAAAGGGCAGCCCCGAGA
ACCACAGGTGTACACCCTGCCCCCATCCCGGGATGAGCTGACCAAGAACCA
GGTCAGCCTGACCTGCCTGGTCAAAGGCTTCTATCCCAGCGACATCGCCGTG
GAGTGGGAGAGCAATGGGCAGCCGGAGAACAACTACAAGACCACGCCTCC
CGTGCTGGACTCCGACGGCTCCTTCTTCCTCTACAGCAAGCTCACCGTGGAC
AAGAGCAGGTGGCAGCAGGGGAACGTCTTCTCATGCTCCGTGATGCATGAG
GCTCTGCACAACCACTACACGCAGAAGAGCCTCTCCCTGTCTCCGGGTAAAT
GA

SEQ ID NO: 38:
MAWVWTLLLLMAAAQSAQAQVKLQQSGAELMKPGASVKISCKATGYTFSRY
WIEWVKQRPGHGLEWIGEILPGSGSTNYNEKFKGKATFTADTSSNTAYMQLSS
LTSEDSAVYYCARGGFLGHYGFAYWGQGTLVTVSAASTKGPSVFPLAPSSKST
SGGTAALGCLVKDYFPEPVTVSWNSGALTSGVHTFPAVLQSSGLYSLSSVVTVP
SSSLGTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGS
FFLYSKLTVDKSRWQQGNVFSCSVMHEALHNHYTQKSLSLSPGK

SEQ ID NO: 39:

ATGAGACCGTCTATTCAGTTCCTGGGGCTCTTGTTGTTCTGGCTTCATGGTGC
TCAGTGTGACATTCAGCTGACCCAGTCTCCAACCACCATGGCTGCATCTCCC
GGGGAGAAGATCACTATCACCTGCAGTGCCAGCTCAAGTATAAGTTCCAAT
TACTTGCATTGGTATCAGCAGAAGCCAGGATTCTCCCCTAAACTCTTGATTT
ATAGGACATCCAATCTGGCTTCTGGAGTCCCAGCTCGCTTCAGTGGCAGTGG
GTCTGGGACCTCTTACTCTCTCACAATTGGCACCATGGAGGCTGAAGATGTT
GCCACTTACTACTGCCAGCAGGGTAGTAGTATACCGTACACGTTCGGAGGG
GGGACCAAGCTGGAGATCAAACGTACGGTGGCTGCACCATCTGTCTTCATC
TTCCCGCCATCTGATGAGCAGTTGAAATCTGGAACTGCCTCTGTTGTGTGCC
TGCTGAATAACTTCTATCCCAGAGAGGCCAAAGTACAGTGGAAGGTGGATA
ACGCCCTCCAATCGGGTAACTCCCAGGAGAGTGTCACAGAGCAGGACAGCA
AGGACAGCACCTACAGCCTCAGCAGCACCCTGACGCTGAGCAAAGCAGACT
ACGAGAAACACAAAGTCTACGCCTGCGAAGTCACCCATCAGGGCCTGAGCT
CGCCCGTCACAAAGAGCTTCAACAGGGGAGAGTGTTAG

SEQ ID NO: 40:
MRPSIQFLGLLLFWLHGAQCDIQLTQSPTTMAASPGEKITITCSASSSISSNYLHW
YQQKPGFSPKLLIYRTSNLASGVPARFSGSGSGTSYSLTIGTMEAEDVATYYCQ
QGSSIPYTFGGGTKLEIKRTVAAPSVFIFPPSDEQLKSGTASVVCLLNNFYPREAK
VQWKVDNALQSGNSQESVTEQDSKDSTYSLSSTLTLSKADYEKHKVYACEVT
HQGLSSPVTKSFNRGEC

### (2) Activity measurement by ELISA

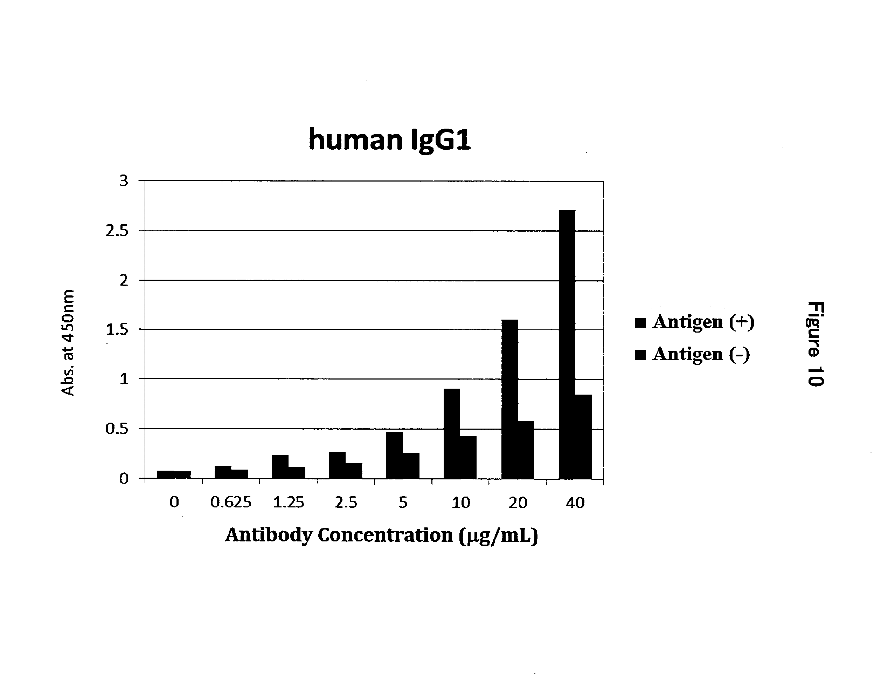

The plasmid prepared in Example 4 was transformed into cells in the same manner as shown in Example 3(2) and (3), followed by purification. The thus prepared human IgG-converted antibody was measured for its activity. In this measurement, the antibody concentration was set to 0 to 40 µg/mL. The results of activity measurement are shown in Figure 10. The human IgG-converted antibody was also found to have activity.

### [Example 5]

In addition to the antibodies described above, newly prepared monoclonal antibodies of subclass IgG were each analyzed by immunostaining in HT-29 and LoVo cells and FACS analysis. 293T cells were transformed with the SLC6A6 gene in the same manner as shown in Example 2(3) to prepare a transiently expressing strain. The cells were collected after 3 days of transformation and stained in the same manner as shown in Example 2(13) for analysis.

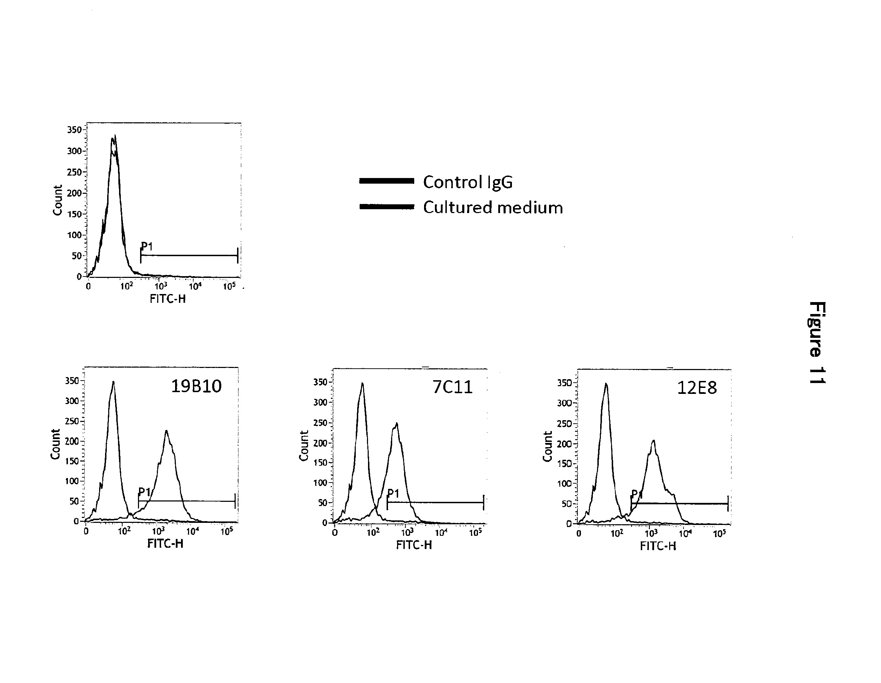

The experimental results obtained are shown in Figure 11. Among the obtained subclass IgG antibodies, at least three clones (19B10, 7C11, 12E8) were found to react with the cells engineered to overexpress SLC6A6.

### [Example 6]

### (1) Antibody-dependent cellular cytotoxicity (ADCC)

Human peripheral blood mononuclear cells were treated with human interleukin-2 for use as effector cells and mixed with the target cells listed below at a ratio of effector cells to target cells = 25:1, 12.5:1, 6.25:1 or 3.13:1, and then cultured for 4 hours (37°C, 5% CO₂) after addition of 0.1 µg/mL humanized anti-SLC6A6 antibody. In the presence of the antibody and the effector cells, ADCC was measured by CytoTox 96 (Promega). This assay is based on the conversion of tetrazolium into formazan by the action of LDH (lactate dehydrogenase) released into the culture solution upon cell death, and this conversion can be detected as a change in the absorbance at 500 nm.
Human colorectal cancer cell line Caco2
Human colorectal cancer cell line HCT15
Human colorectal cancer cell line DLD-1
Human colorectal cancer cell line HCT116
Human colorectal cancer cell line LoVo
Human colorectal cancer cell line SW480
Human colorectal cancer cell line SW620
Human colorectal cancer cell line WiDr

### [Example 7]

### (1) Complement-dependent cellular cytotoxicity (CDC)

To HCT15 cells (SLC6A6-positive cells derived from human colorectal cancer), either 25 µg/mL anti-SLC6A6 antibody or 25 µg/mL chimerized anti-SLC6A6 antibody was added. To this, 10 µg/mL fluorescein-labeled human complement C1q was added. The level of fluorescent staining with the labeled C1q was measured by flow cytometry. Next, to HCT15 cells or HT-29 cells (SLC6A6-positive cells) and COLO201 cells (weakly SLC6A6-positive cells), 2.2 µg/mL anti-SLC6A6 antibody was added. To this, human complement was added and cultured. The amount of LDH released into the extracellular environment was measured by CytoTox 96 to calculate the rate of cytolysis caused by the complement.

### [Example 8]

### (1) Inhibitory effect on the number of SLC6A6 (Internalization)

Human colorectal cancer cells HCT15 (SLC6A6-hyperexpressing cell line) and COLO201 (SLC6A6-hypoexpressing cell line) were cultured in the presence or absence of 150 µg/mL anti-SLC6A6 antibody for 1 day or 5 days (37°C, 5% CO₂), followed by determining the number of SLC6A6 in these cells. To determine the number of SLC6A6 per cell, AlexaFluor 488-labeled anti-SLC6A6 monoclonal antibody 4B9b was used, and the molar absorption coefficient of AlexaFluor 488 was used to calculate the number of SLC6A6 per cell.

### [Example 9]

### (1) Preparation of cell lines

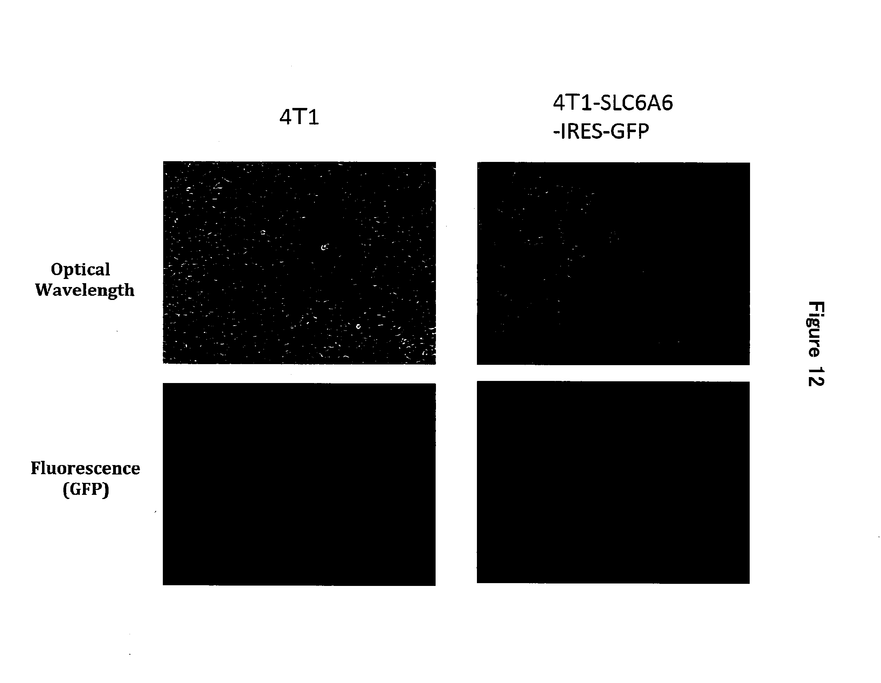

IRES and the GFP gene were introduced downstream of SLC6A6 which had been introduced into pEF6 vector in Example 2(3). The IRES-GFP fragment was prepared as follows: An IRES-GFP segment was cleaved from pMXs-IG vector (purchased from Dr. Toshio Kitamura, the University of Tokyo) with restriction enzymes (XhoI and SalI) and blunt-ended with Klenow fragment (Takara Bio Inc., Japan). Likewise, the pEF6-SLC6A6 vector prepared in Example 2(2) was cleaved downstream of SLC6A6 with a restriction enzyme (PmeI) and also blunt-ended with Klenow fragment. The IRES-GFP fragment was cloned into the blunt-ended pEF6-SLC6A6 vector to construct pEF6-SLC6A6-IRES-GFP vector. This plasmid was transformed into mouse breast cancer cells 4T1 using Fugene 6. Transformation was accomplished in the same manner as shown in Example 2(3). A plurality of cells showing GFP fluorescence were cloned and observed for their morphology, indicating that these cells were morphologically easy to float when compared to non-transformed cells (Figure 12).

### (2) Phenotype analysis

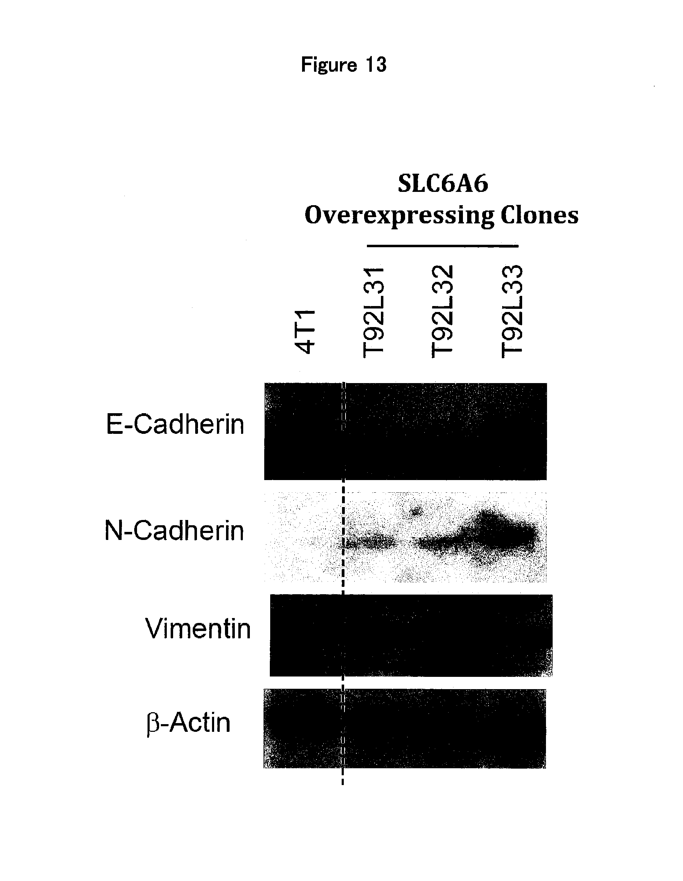

The cells engineered to overexpress SLC6A6 were cultured and analyzed by Western blot in the same manner as shown in Example 2(4). In this case, anti-E-cadherin antibody, anti-N-cadherin antibody and anti-Vimentin antibody (BD) were each used at 1 µg/mL. The experimental results obtained are shown in Figure 13. A change in phenotype as seen in epithelial-mesenchymal transition (EMT) was observed. Namely, the results suggested a possibility that SLC6A6 would not only transport taurine, but would also be responsible for turning cancer cells into cancer stem cells upon overexpression.

### [Example 10]

### (1) Effect on Balb/c mouse transplantable SLC6A6 cell line 4T1-SLC6A6

The mouse IgG-converted antibody prepared in Example 3 was analyzed for its *in vivo* effect on the 4T1 cells prepared to overexpress SLC6A6 in Example 9. The cells established after transformation, i.e., L31 and L35 were each transplanted (5 × 10⁵ cells) into mammary glands in the same manner as shown in Example 2(5). At 13 days after transplantation, the mouse IgG-converted antibody was administered at 50 µg/kg, and the tumor size (diameter) was measured at 0, 7, 12 and 15 days after administration.

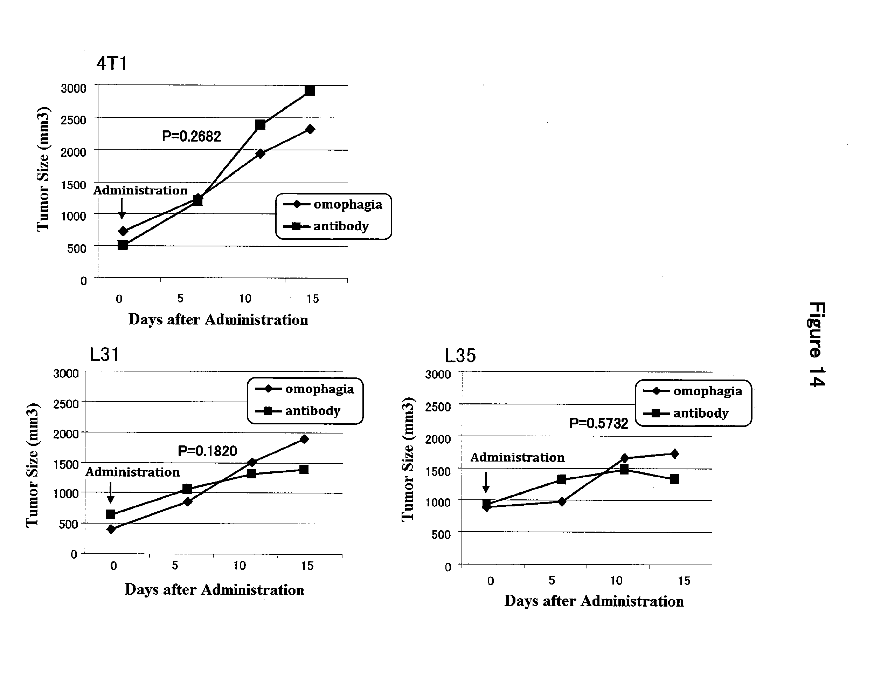

The results obtained are shown in Figure 14, which indicate that the SLC6A6-expressing cells are prevented from proliferating by the action of the added antibody.

### [Example 11]

For more detailed analysis of the results obtained in Example 10, cells of mouse breast cancer 4T1-SLC6A6 (SLC6A6-transformed cell line) were transplanted into the fourth mammary glands of nude mice (5 to 7 mice per group, female), and antibody administration was started at 7 days after transplantation. The anti-SLC6A6 antibody (total dose: 0.3 to 100 mg/kg) was administered via the tail vein once a day on days 1, 5 and 9 (day of starting administration = day 1), i.e., three times in total. The control group was administered via the tail vein with mouse IgG1 (total dose: 100 mg/kg) in the same schedule.

### (2) Effect on nude mouse transplantable human colorectal cancer cell line HCT15 Anti-SLC6A6 antibody alone

Cells of human colorectal cancer HCT15 (SLC6A6-hyperexpressing cell line) were transplanted under the dorsal skin of nude mice (5 to 10 mice per group, female) and started to receive administration at the time point when the tumor volume reached 200 to 300 mm³. The anti-SLC6A6 antibody (0.1 to 30 mg/kg/day) was administered intraperitoneally once a day, twice a week for 4 to 5 weeks. The control group was intraperitoneally administered with human IgG1 in the same schedule.

### (3) Anti-SLC6A6 antibody + taxol

Cells of human colorectal cancer HCT15 (SLC6A6-hyperexpressing cell line) were transplanted under the dorsal skin of nude mice (7 to 13 mice per group) and started to receive administration at the time point when the tumor volume reached 200 to 300 mm³. The tumor volume was measured to examine antitumor effects.

### [Example 12]

### (1) Cell proliferation test

Cells of human colorectal cancer HCT15 (SLC6A6-hyperexpressing cell line) were used and cultured in the presence of anti-SLC6A6 antibody for 3 days (37°C, 5% CO₂).

### [Example 13]

### (1) Preparation of SLC6A6-overexpressing cell lines of human colorectal cancer cells

pEF6-IRES-GFP prepared in Example 10(1) was transformed into COLO201, SW620, HT-29 and LoVo cells, followed by drug selection with 10 µg/mL blasticidin to obtain stably expressing cell lines.

### [Example 14]

### (1) Preparation of RNAi vectors

Sequences were designed to suppress SLC6A6 expression by RNAi. For this purpose, sequence design software siDirect version 2.0 (http://sidirect2.rnai.jp/) was used. To obtain cells where expression was stably suppressed, four types of sequences were designed to construct shRNAs. Two synthetic oligo DNAs were designed to comprise a complementary sequence and they were annealed to each other (denaturation at 94°C for 10 minutes, followed by lowering the temperature to room temperature over 12 hours) to thereby prepare a fragment to be inserted into a plasmid vector. The fragment was then cloned into pCAG vector (obtained from Dr. Murakami's laboratory, Tokyo University of Science) by means of the restriction enzymes (EcoRI and BamHI) designed on the primers.

The thus prepared four shRNA sequences are shown in SEQ ID NOs: 41, 44, 47 and 50, respectively. The primer sequences used to prepare the respective sequences are as follows: the primers shown in SEQ ID NOs: 42 and 43 were used for preparation of the sequence shown in SEQ ID NO: 41, the primers shown in SEQ ID NOs: 45 and 46 were used for preparation of the sequence shown in SEQ ID NO: 44, the primers shown in SEQ ID NOs: 48 and 49 were used for preparation of the sequence shown in SEQ ID NO: 47, and the primers shown in SEQ ID NOs: 51 and 52 were used for preparation of the sequence shown in SEQ ID NO: 50.
SLC6A6-shRNA1: GCTGCAGTGTCTGAAAGATTT (SEQ ID NO: 41)
Forward primer: 
GTTTGCTGCAGTGTTTGAAAGATTTGTGTGCTGTCCAAATCTTTCAGACACT
GCAGCTTTTTCGACGTCACAAACTTTCTAAACACACGACAGGTTTAGAAAGT
CTGTGACGTCGAAAAACGTA (SEQ ID NO: 42)
 Reverse primer: 
ATGCAAAAAGCTGCAGTGTCTGAAAGATTTGGACAGCACACAAATCTTTCA
AACACTGCAGC (SEQ ID NO: 43)
 SLC6A6-shRNA2: GCGTTTCTCATACCGTATT (SEQ ID NO: 44) Forward primer: 
GTTTGCGTTTCTCATACTGTATTGTGTGCTGTCCAATACGGTATGAGAAACG
CTTTTTCGCAAAGAGTATGACATAACACACGACAGGTTATGCCATACTCTTT
GCGAAAAACGTA (SEQ ID NO: 45)
 Reverse primer: 
ATGCAAAAGCGTTTCTCATACCGTATTGGACAGCACACAATACAGTATGA
GAAACGC (SEQ ID NO: 46)
 SLC6A6-shRNA3: GCTATGCCTCCGTTGTAATTG (SEQ ID NO: 47) Forward primer: 
GTTTGCTATGCCTCTGTTGTAATTGGTGTGCTGTCCCAATTACAACGGAGGC
ATAGCTTTTTCGATACGGAGaCAACATTAACCACACGACAGGGTTAATGTTG
CCTCCGTATCGAAAAACGTA (SEQ ID NO: 48)
 Reverse primer: 
ATGCAAAAAGCTATGCCTCCGTTGTAATTGGGACAGCACACCAATTACAAC
AGAGGCATAGC (SEQ ID NO: 49)
 SLC6A6-shRNA4: GACCTACAACAAAACATACGT (SEQ ID NO: 50) Forward primer: 
GTTTGACCTACAACAAAATATACGTGTGTGCTGTCCACGTATGTTTTGTTGT
AGGTCTTTTTCTGGATGTTGTTTTATATGCACACACGACAGGTGCATACAAA
ACAACATCCAGAAAAACGTA (SEQ ID NO: 51)
 Reverse primer:

ATGCAAAAGACCTACAACAAAACATACGTGGACAGCACACACGTATATTT
TGTTGTAGGTC (SEQ ID NO: 52)

### (2) Suppression of SLC6A6 expression in colorectal cancer cells

Colorectal cancer cells HCT15 and HT-29 were transformed with the vectors constructed in Example 10(1) in the same manner as shown in Example 2(3). Lipofectamine RNAi MAX (Invitrogen) was used as a transformation reagent and drug selection was conducted in a medium containing 10 µg/mL blasticidin to obtain cell lines. Whether SLC6A6 expression was suppressed was analyzed by Western blot in the same manner as shown in Example 2(4).

### (3) Test for antibody-induced suppression of migration ability (membrane migration assay)

The cells prepared in Example 9 were suspended in a serum-free medium and then introduced into a Control Culture Insert (BD) transwell (upper side of filter: upper compartment) at 1 × 10⁵ cells, and further supplemented with anti-SLC6A6 antibody or mouse IgG1 antibody at 50 µg/well, followed by culture at 37°C for 3 days. Cells which had migrated through the membrane (pore size: 8 µm) in the transwell (lower side of filter: lower compartment) were stained with a 0.1% crystal violet solution, washed with Milli-Q water to remove excess dye and then observed for cell counts.

### [Example 15]

### (1) Test for antibody-induced suppression of invasion ability (Matrigel permeability assay)

The cells prepared in Example 11 were suspended in a serum-free medium and then introduced into a Matrigel Invasion Chamber (BD) transwell (upper side of filter: upper compartment) at 1 × 10⁵, and further supplemented with anti-SLC6A6 antibody or mouse IgG1 antibody at 50 µg/well, followed by culture at 37°C for 3 days. Cells which had migrated through the membrane (pore size: 8 µm) in the transwell (lower side of filter: lower compartment) were stained with a 0.1 % crystal violet solution, washed with Milli-Q water to remove excess dye and then observed for cell counts.

### [Example 16]

### (1) Test for antibody-induced suppression of migration ability (scratch assay)

The cells prepared in Example 11 were cultured in plastic plates and the cultured surface was scratched. Culture was continued for 12 hours in a medium supplemented with or without anti-SLC6A6 antibody, and the rate of change in the scratched area was measured to determine the motility for each cell.

### [Example 17]

### (1) Colony formation test

The cells prepared in Example 11 were analyzed with a CytoSelecto Cell Transformation Assay Kit (CELL BIOLABS). The cells were dispensed at 1 × 10² cells/well in soft agar and cultured for two weeks, and the colonies formed were then counted.

### INDUSTRIAL APPLICABILITY

The present invention provides a monoclonal antibody which specifically binds to the extracellular region of SLC6A6. Moreover, the present invention also provides a nucleic acid which suppresses SLC6A6 expression. The antibody of the present invention can be used for cancer treatment through specifically binding to SLC6A6-expressing cancer cells. The nucleic acid which suppresses SLC6A6 expression can inhibit the proliferation of SLC6A6-expressing cancer cells and the progression of their metastasis.

### Deposition Numbers

(1) Microorganism is labeled as: "Mouse-Mouse hybridoma 4B9b"
   Accession No.: FERM BP-11413
   Initial deposit date: July 21, 2010
   International Deposition Authority:
   International Patent Organism Depositary, the National Institute of Advanced Industrial Science and Technology
   Central 6, 1-1-1 Higashi, Tsukuba-shi, Ibaraki 305-8566, Japan
(2) Microorganism is labeled as: "mouse-mouse hybridoma 5H12d"
   Accession No.: FERM BP-11414
   Initial deposit date: July 21, 2010
   International Deposition Authority:
   International Patent Organism Depositary, the National Institute of Advanced Industrial Science and Technology
   Central 6, 1-1-1 Higashi, Tsukuba-shi, Ibaraki 305-8566, Japan

### Sequence Listing Free Text

SEQ ID NOs: 5-20, 25-36 and 41-52: synthetic DNAs

## Claims

1. A pharmaceutical composition comprising a monoclonal antibody or a fragment thereof, which recognizes native SLC6A6, or a substance capable of suppressing SLC6A6 expression by RNA interference.

2. A pharmaceutical composition comprising a monoclonal antibody or a fragment thereof, which recognizes a polypeptide in the extracellular region of SLC6A6.

3. The pharmaceutical composition according to claim 2, wherein the polypeptide in the extracellular region is at least one selected from (a) to (c) shown below:
(a) a polypeptide which consists of the amino acid sequence shown in SEQ ID NO:4;
(b) a polypeptide which consists of an amino acid sequence with substitution, deletion and/or insertion of one or several amino acids in the amino acid sequence shown in SEQ ID NO: 4 and which serves as an extracellular region of SLC6A6; and
(c) a polypeptide which consists of an amino acid sequence sharing a homology of 70% or more with the amino acid sequence shown in SEQ ID NO: 4 and which serves as an extracellular region of SLC6A6.

4. A pharmaceutical composition comprising a monoclonal antibody against SLC6A6 or a fragment thereof, which is produced by a hybridoma having Accession No. FERM BP-11413 or FERM BP-11414.

5. A pharmaceutical composition comprising a monoclonal antibody against SLC6A6 or a fragment thereof, which binds to an epitope recognized by the monoclonal antibody or fragment thereof according to claim 4.

6. The pharmaceutical composition according to any one of claims 1 to 5, wherein the monoclonal antibody is human or humanized.

7. The pharmaceutical composition according to any one of claims 1 to 5, wherein the monoclonal antibody is fused.

8. The pharmaceutical composition according to any one of claims 1 to 7, which is intended for treatment of colorectal cancer.
